# EUROPEAN PATENT SPECIFICATION

(11) **EP 1 663 283 B1**
(45) Date of publication and mention of the grant of the patent: **23.05.2012**
(21) Application number: 04781267.2
(22) Date of filing: 16.08.2004
(51) Int. Cl.: A61K 38/00, A61K 38/05

(54) **GLUTAMINE FOR USE IN TREATING INJURY**
GLUTAMIN ZUR VERWENDUNG BEI DER BEHANDLUNG VON VERLETZUNGEN
GLUTAMINE POUR LE TRAITEMENT DE LESION

(30) Priority: 12.09.2003 US 502574 P; 28.01.2004 US 539646 P
(43) Date of publication of application: 07.06.2006
(73) Proprietor: University of Colorado, Boulder, CO 80309 (US)
(72) Inventor: WISCHMEYER, Paul, University of Colorado, Boulder, CO 80309 (US); SINGLETON, Kristen, University of Colorado, Boulder, CO 80309 (US)
(74) Representative: Richly, Erik
(86) International application number: PCT/US2004/026551
(87) International publication number: WO 2005/034980

(56) References cited:
- EP-A- 0 087 750
- JP-A- 8 295 633
- US-A- 5 543 397
- US-A- 5 561 111
- US-A- 5 786 337
- US-A- 5 849 335
- US-A- 5 984 590
- DATABASE EPODOC EUROPEAN PATENT OFFICE, THE HAGUE, NL; XP002485398 -& SK 283 178 B6 (VYZK USTAV ANTIBIOTIK A BIOTRA [CZ] INFUSIA A S [CZ]) 4 March 2003 (2003-03-04)
- DATABASE MEDLINE [Online] US NATIONAL LIBRARY OF MEDICINE (NLM), BETHESDA, MD, US; July 1999 (1999-07), WILMORE D W ET AL: "Glutamine in the support of patients following bone marrow transplantation." XP002484419 Database accession no. NLM10453314 & CURRENT OPINION IN CLINICAL NUTRITION AND METABOLIC CARE JUL 1999, vol. 2, no. 4, July 1999 (1999-07), pages 323-327, ISSN: 1363-1950
- DATABASE BIOSIS [Online] BIOSCIENCES INFORMATION SERVICE, PHILADELPHIA, PA, US; 2 April 1998 (1998-04-02), GORINGE A P ET AL: "Glutamine and vitamin E in the treatment of hepatic veno-occlusive disease following high-dose chemotherapy" XP002484420 Database accession no. PREV199800259803 & BONE MARROW TRANSPLANTATION, vol. 21, no. 8, 2 April 1998 (1998-04-02), pages 829-832, ISSN: 0268-3369
- DATABASE BIOSIS [Online] BIOSCIENCES INFORMATION SERVICE, PHILADELPHIA, PA, US; 2002, HOLECEK M ET AL: "Effect of alanyl-glutamine on leucine and protein metabolism in irradiated rats" XP002484421 Database accession no. PREV200200304391 & AMINO ACIDS (VIENNA), vol. 22, no. 1, 2002, pages 95-108, ISSN: 0939-4451
- OEHLER ET AL.: 'Glutamine depletion impairs cellular stress response in human leucocytes' BRITISH JOURNAL OF NUTRITION vol. 87, no. SUPP.1, 2002, pages S17 - S21, XP002983430

## Description

### BACKGROUND OF THE INVENTION

### 1. Technical Field

The present invention relates to the treatment of injury. More specifically, the present invention relates to the use of glutamine for treating injury.

### 2. Description of the Related Art

Nutritional therapies are commonly applied in ill people in order to enhance physical capacity and recovery from stresses due to medical conditions. Many times the recommendations simply include dietary advice regarding the distribution of carbohydrates, proteins, and fats in the overall diet. A more advanced approach is to recommend supplementation of key nutrients that will aid healing and enhance the physical state of the individual. Such nutritional formulations may be termed "dietary supplements," "functional foods" or "medical foods." In order to formulate an effective dietary supplement or functional or medical food, an understanding of the scientific basis behind the key ingredients is essential. Once a well-grounded recommendation toward dietary modification is made it can have a powerful influence on the rate of recovery in the individual who is in poor health.

Often, persons who consider themselves to be in good health with a good nutritional status are actually somewhat suboptimal in both parameters, rendering them at risk for developing such medical conditions. Dietary supplements, functional or medical foods developed for improving cardiovascular function may also benefit such persons as cardioprotectors.

In the area of medically recommended supplementation, artificial diets have played a key role for many years. Post-surgery, the gastro-intestinal tract of a patient is typically unable to properly digest food for several days. In such cases parenteral nutrition is essential, wherein the patient is given glucose or a carefully formulated mixture of salts, carbohydrates, amino acids, fatty acids, and vitamins. Even after the patient is weaned from parenteral nutrition, enteral nutrition with a similar composition may be established orally or via a feeding tube, or a medical food enteral supplement may be added to his or her diet in order to optimize the types and amounts of nutrients the patient requires and receives.

The most pressing need for improved prevention, rehabilitation and maintenance regimens is in the area of cardiovascular disease, which is the leading cause of death worldwide. It has been projected that one of five persons in the United States has cardiovascular disease. Within this arena, myocardial infarction accounts for more than half a million deaths per year. Furthermore, survivors face a level of morbidity and subsequent disability that affects their medical, social, and of equal importance, economic status. Therefore, surviving the initial acute event of a myocardial infarction leaves patients with a variety of challenges. Such patients may be left in a state of compromised cardiovascular function such as chronic ischemic disease, congestive heart failure or reduced peripheral blood flow.

Congestive heart failure may have a more insidious onset than that following myocardial infarction. Atherosclerosis may gradually lessen circulation to the heart or uncontrolled hypertension may weaken the heart muscle. Another condition, cardiomyopathy, may occur from a variety of causes including ischemia, hypertension or chronic infection. Whatever the cause, these types of cardiovascular disease may present a similar clinical picture and pose the same problems of treatment and maintenance as does myocardial infarction.

Peripheral vascular disease is closely related to cardiovascular disease, in that the same underlying cause, atherosclerosis, may impair circulation to the skeletal muscles, brain or kidneys, interfering with their function. A nutritional supplement that benefits subjects with cardiovascular disease will also benefit these subjects.

Over the past twenty years, cardiac rehabilitation has provided survivors with an increased quality of life. Cardiac rehabilitation programs have continued to change to meet the needs and expectations of these afflicted individuals. An important aspect of successful rehabilitation is a gradual programmed increase in exercise training with an attention to modifying existing cardiac risk factors. The ultimate goal in any cardiac rehabilitation program is the improvement of functional capacity, the lessening of awareness of activity-produced symptoms, the reduction of disability and the modification of known coronary risk factors for the prevention of subsequent cardiovascular events, that is, to provide cardioprotection. Many patients feel strongly that a good quality of life includes the ability to resume their pre-disease activity, if at all possible.

While general nutritional supplementation is the standard mode of therapy as part of a disease management program, a more focused nutritional program can have more specific and powerful benefits. For example glutamine is useful in the treatment of diseases of the liver due to its ability to increase blood flow to the liver (United States Patent No. 6,001,878). Glutamine is also effective at maintaining the immune system. This was shown in a study where there was a lower level of infection in patients following bone marrow transplantation when their parenteral nutritional program was supplemented with glutamine (Calder and Yapoob 1999). Another example is taurine which has a positive inotropic effect on the heart and can be used as a treatment in congestive heart failure. In a clinical trial 4 weeks of taurine supplementation led to a highly significant improvement in dyspnea, palpitation, crackles, edema, and New York Heart Association functional class (Azuma et al 1983). However, there has been no disclosure of how to use glutamine to prevent heart damage or potential uses of glutamine for preventing or treating other problems.

Myocardial ischemia/reperfusion injury (I/R) is one of the most important, potentially reversible stressors during the peri-operative period. Due to lack of a successful therapeutic regimen, pre-operative prophylaxis against myocardial I/R injury is not a clinically routine procedure. This is particularly relevant in procedures requiring cardiopulmonary bypass, such as coronary artery bypass grafting (CABG) where myocardial I/R injury is an unavoidable consequence. Two of the major mechanisms by which protection of the myocardium against I/R injury can be achieved include: 1) stimulation of stress protein (i.e. heat shock proteins) synthesis and 2) prevention of adenosine triphosphate (ATP) depletion and a limitation of the acidosis during prolonged ischemia.

Glutamine (GLN), traditionally considered a non-essential amino acid, now appears to be a conditionally essential nutrient during serious injury or illness. Serious illness and injury has shown an increased utilization of GLN by the gut, inflammatory cells, and the kidney. These rapidly replicating cells selectively consume glutamine for its essential role in nucleic acid synthesis and as a preferential fuel during stress. In good health, GLN is the most abundant amino acid in plasma and skeletal muscle, but circulating and tissue concentrations fall precipitously after injury, surgery, or infection. Specific to CABG procedures in human patients, previous research showed that circulating plasma GLN levels fall by at least 30 % post CABG. Additional data indicates that myocardial tissue GLN also falls significantly post- cardiopulmonary bypass in patients undergoing CABG.

Extensive research indicates that GLN supplementation can be protective against cellular and organ injury *in vitro* and *in vivo.* Further, it is likely that the fall in plasma GLN following severe illness or injury is likely detrimental to the function of multiple organs including the heart. Preliminary studies in human subjects have demonstrated that GLN can improve outcomes in a number of patient populations. Important mechanisms of GLN's protective effects in the myocardium can relate to enhancement of tissue heat shock protein synthesis, support of tissue metabolism, and preservation of tissue redox state.

Heat Shock Proteins (HSP) are a family of highly conserved proteins involved in the most basic mechanisms of cellular protection. The expression of these proteins following a sublethal insult can induce "stress tolerance", and provide protection from a subsequent stress that would otherwise be lethal. Experimental data has shown that preinduction of the heat stress response can provide marked protection against many form of cellular injury, including ischemia/reperfusion injury. The role of HSPs, specifically HSP 72 (72 refers to the molecular weight of the protein), in protection of the myocardium from ischemia/reperfusion (I/R) injury has been extensively described. Specifically, genetic manipulation, leading to overexpression of HSP 72 has been shown to limit the detrimental effects of (I/R) injury in mice. These findings indicate that enhanced HSP 72 expression can confer significant protection against I/R injury. The induction of HSPs to improve outcome in human disease has not been exploited because induction agents utilized in the laboratory are themselves toxic and not clinically relevant. Agents presently used to enhance HSP expression in the laboratory include acute hyperthermia and heavy metals, such as sodium arsenite. These methods of HSP 72 enhancement have significant toxicity associated with their use and therefore a clinically relevant method of enhancing HSP 72 expression to protect myocardial tissue against I/R injury would be desirable.

It would therefore be useful to develop a therapeutic for the treatment and prevention of heart damage and other cellular damage that do not have the detrimental side effects disclosed above. It would also be beneficial to develop a manner of increasing the production of HSPs *in vivo* without having to administer HSPs to a patient.

JP8295633 is directed to a a complication-preventing agent for patients in high protein catabolismic state. The document available as DATABASE EPODOC EUROPEAN PATENT OFFICE, THE HAGUE, NL, XP002485398 discloses treatment of sepsis, polytraumas and post-operative states in which there is a metabolic breakdown by infusions containing the alanyl-L-glutamine dipeptide and various amino acids. The document available as DATABASE MEDLINE [Online] US NATIONAL LIBRARY OF MEDICINE (NLM), BETHESDA, MD, US; July 1999 (1999-07), WILMORE D W ET AL: "Glutamine in the support of patients following bone marrow transplantation." XP002484419 Database accession no. NLM10453314 discloses the use of parenteral/intravenous (or enteral) glutamine dipeptide for patients undergoing cytotoxic chemotherapy and irradiation resulting in severe catabolism and disruption of intestinal mucosa following bone marrow transplantation. The document available as DATABASE BIOSIS [Online] BIOSCIENCES INFORMATION SERVICE, PHILADELPHIA, P.A, US; 2 April 1998 (1998-04-02), GORINGE A P ET AL: "Glutamine and vitamin E in the treatment of hepatic veno-occlusive disease following high-dose chemotherapy" discloses the use of infusions of glutamine (as dipeptide) for the treatment of hepatic veno-occlusive disease of the liver which is a comon complication following bone marrow transplantation. The document available as DATABASE BIOSIS [Online] BIOSCIENCES INFORMATION SERVICE, PHILADELPHIA, PA, US; 2002, HOLECEK M ET AL: "Effect of alanyl-glutamine on leucine and protein metabolism in irradiated rats" XP002484421 Database accession no. PREV200200304391 discloses the use in rats of intragastrally fed alanyl-glutamine to improve disturbances caused by irradiation. EP0087750 relates to amino acid preparations containing glutaminic residue. The publication by OEHLER ET AL. ('Glutamine depletion impairs cellular stress response in human leucocytes' BRITISH JOURNAL OF NUTRITION vol. 87, no. SUPP.1, 2002, pages S17 - S21) describes that the major inducible heat shock protein HSP70 is improved by high glutamine concentrations. US5849335 discloses that in episodes of stress such as sepsis, injury, burns, inflammation, diarrhoe and surgery there is a marked increase in glutamine consumption leading to glutamine deficiency.

### SUMMARY OF THE INVENTION

The invention relates to a di-peptide said di-peptide being selected from the group consisting of alanyl-glutamine, glycyl-glutamine, leucyl-glutamine, valyl-glutamine, isoleucyl-glutamine, and cysteinyl-glutamine for use in the treatment or prevention of an acute or chronic illness, disease, or injury; the prevention of ischemia or reperfusion injury; the protection of organs for transplantation; attenuating inflammation or pro-inflammatory cytokine expression in states of health or disease; the prevention or treatment of heat stress, heat stroke, or any other temperature related stress or injury; improving tissue metabolism; and treating sepsis; wherein the illness, disease or injury is one of lung injury, acute respiratory distress syndrome, an autoimmune illness; arthritis, osteoarthritis, rheumatoid arthritis, Lupus, multiple sclerosis, fibromyalgia, Chrohn's disease, ulcerative colitis, irritable bowel syndrome, a heart attack, cardiac surgery, chronic angina, coronary artery disease, peritonitis and systemic inflammatory response syndrome, wherein the medicament is arranged to be delivered in an intravenous bolus dose.

Furthermore, the invention relates to a di-peptide said di-peptide being selected from the group consisting of alanyl-glutamine, glycyl-glutamine, leucyl-glutamine, valyl-glutamine, isoleucyl-glutamine, and cysteinyl-glutamine for use in the treatment of local and systemic inflammatory diseases and autoimmune conditions, in order to protect organs for transplantation, wherein the medicament increases the expression of any heat shock protein or heat shock factor 1 and wherein the medicament is arranged to be delivered in an intravenous bolus dose.

The invention is also directed to a di-peptide said di-peptide being selected from the group consisting of alanyl-glutamine, glycyl-glutamine, leucyl-glutamine, valyl-glutamine, isoleucyl-glutamine, and cysteinyl-glutamine for use in inhibiting skin/epithelial damage due to UV radiation, bum injury, aging, or any other type of radiation injury, wherein the medicament increases the expression of any heat shock protein or heat shock factor 1 and wherein the medicament is arranged to be delivered in a single intravenous bolus dose. The invention also relates to a di-pepitde said di-peptide being selected from the group consisting of alanyl-glutamine, glycyl-glutamine, leucyl-glutamine, valyl-glutamine, isoleucyl-glutamine, and cysteinyl-glutamine for use in inhibiting skin or mucosal injury from chemotherapy or therapeutic radiation, wherein the medicament increases the expression of any heat shock protein or heat shock factor 1 and wherein the medicament is arranged to be delivered as an oral, a topical or an intravenous bolus dose.

### DESCRIPTION OF THE DRAWINGS

Other advantages of the present invention are readily appreciated as the same becomes better understood by reference to the following detailed description, when considered in connection with the accompanying drawings wherein:
Figure 1 is a flow chart showing mechanisms of glutamine's benefits on myocardial/reperfusion injury in coronary artery bypass patients;
Figures 2A and B show the dose response effect of GLN on HSP 72 expression in heart and lung tissue in unstressed rats;
Figures 3A and B show the effect of GLN on HSP 72 expression in hearts of LPS-injured rats;
Figure 4 is a graph showing the effect of Glutamine (GLN) on cell death from ischemia/reperfusion;
Figures 5A and B are graphs showing the effect of glutamine (GLN) pretreatment on cardiac indices following: ischemia/reperfusion injury in rat working heart model;
Figure 6 is a graph showing the effect of myocardial ischemia/reperfusion injury on myocardial tissue ATP/ADP ratio in rat working heart model;
Figure 7 is a graph showing the effect of myocardial ischemia/reperfusion injury on myocardial tissue lactate in rat working heart model;
Figure 8 is a graph showing the effect of myocardial ischemia/reperfusion injury on myocardial tissue substrate (glutamate, glutamine, and glucose) in rat working heart model;
Figure 9 is a graph showing the effect of myocardial ischemia/reperfusion injury on myocardial tissue reduced glutathione content in rat working heart model;
Figure 10 is a graph showing the effect of myocardial ischemia/reperfusion injury on myocardial tissue NAD(+)/NADH content in rat working heart model;
Figure 11 is ¹H-MRS spectra from rat myocardial tissue exposed to ischemia and reperfusion injury in rat working heart model;
Figure 12 shows the pathway by which glutamine protects intestinal epithelial cells via inductions of HSP 72;
Figures 13A and B show that glutamine induces HSP 70 in heart and lung tissue;
Figure 14 is a graph showing that glutamine enhances survival from LPS injury;
Figure 15 is a photograph showing that glutamine enhances HSP 70 expression in tissues of LPS-injured rats;
Figures 16A and B are graphs showing the effect of GLN on TNF-α and IL-1β release from LPS injection;
Figure 17 is a graph showing that GLN enhances lung HSP 70 expression;
Figure 18 is a graph showing that GLN enhances lung HSF-1 activation; ,
Figure 19 is a graph showing that glutamine improves high-energy phosphate content following CLP;
Figure 20 is a graph showing that glutamine improves high-energy phosphate content following CLP;
Figure 21 is a graph showing that glutamine improves survival post-cecal ligation and puncture;
Figure 22 is a graph showing cardiac output versus reperfusion;
Figure 23 is a graph showing that glutamine enhances myocardial glutathione content post-I/R injury;
Figure 24 is a graph showing that oral glutamine improves survival in the rat model of experimental heat stroke;
Figure 25 is a graph showing serum HSP 70 expression in critically ill patients following GLN treatment;
Figure 26 is a graph showing HSP-70 levels at 1-week in patients who received Glutamine compared to control patients;
Figure 27 is a graph showing HSP-70 levels at 1-week in patients who received Glutamine compared to control patients;
Figure 28 is a graph showing HSP-70 levels at 1-week in patients who received Glutamine compared to control patients; and
Figure 29 is a graph showing HSP-70 levels at 1-week in patients who received Glutamine compared to control patients.

### DESCRIPTION OF THE INVENTION

Generally, a treatment of injury or disease utilizing large doses of glutamine (GLN), a conditionally essential amino acid is disclosed. More specifically, a single bolus dose treatment of GLN in a pharmaceutically acceptable carrier for either preventing or treating injury or disease by administering to a patient in need is disclosed.

A therapeutic including a single bolus dose of glutamine can be used for treating acute illness, chronic illness, disease, and injury. Examples of acute illnesses, diseases, and injuries include, but are not limited to, ischemia (both heart and kidney), transplantation, sepsis, hypothermia, lung injury, local and systemic inflammatory diseases, autoimmune conditions, heat shock, heat stroke, and reperfusion injury. The therapeutic functions based upon glutamine's ability to prevent metabolic dysfunction following ischemia and reperfusion and that GLN is an INOS and eNOS inhibitor. This sort of glutamine dosing can improve outcomes from Cardiac diseases such as coronary artery disease, heart attacks, and cardiopulmonary bypass procedures. Additionally, the therapeutic can be useful in treating other problems including, but not limited to, diseases and injuries associated with tissue metabolism and for increasing heat shock protein or heat shock factor-1 expression *in vivo.*

Glutamine can also be utilized in inhibiting or treating skin/epithelial/mucosal damage. Such damage can occur as a result of UV radiation, burn injury, aging, chemotherapy, therapeutic radiation or other form of radiation injury.

The glutamine can be administered to substantially increase the production of heat shock protein (HSP). The GLN, can be used for treating and preventing heat related Illnesses such as, but not limited to, heat stress and heat stroke.

Further, GLN administered either orally or in another form, can have an anti-inflammatory/anti-cytokine effect and thus can be useful In treating inflammatory Illnesses such as, but not limited to, autoimmune disorders such as arthritis (including osteoarthritis and rheumatoid arthritis), Lupus, fibromyalgia, and other related autoimmune diseases, Chrohn's disease, irritable bowel syndrome, and other diseases that result in inflammation or increased production of cytokines.

Additionally, GLN administered either orally or in another form, can be organ-protective. In other words, GLN can be administered to an individual and if organs are removed from the individual, the organs are able to remain viable for an extended period of time outside of the body of the individual. Further, GLN can be administered to an organ, tissue, or cell to provide the organ, tissue, or cell with protection. The benefit of such a property is that if organs can survive a longer period of time after being harvested, then there is a greater likelihood that the organs can be donated to another individual in need of the same. Examples of such organs that can be protected include, but are not limited to, liver, heart, lungs, kidney, and other harvestable organs. Similarly, GLN can be used can be utilized as a portion of a resuscitation fluid or organ transplant preservation solution in combination with other electrolytes. Examples of such fluids/solutions include, but are not limited to, Ringer's lactate solution and other similar solutions known to those of skill in the art.

Glutamine is administered as alanyl-glutamine, glycyl-glutamine, leucyl-glutamine, valyl-glutamine, isoleucyl-glutamine, and cysteinyl-glutamine. The dipeptide can solubilize easily and clears through the body within a few hours, thus eliminating a problem with regard to toxicity. The peptide can also include an acylated terminal nitrogen. Preferably, the acylating moiety for the acylated terminal nitrogen is acetyl.

The dosage of glutamine is based upon normal dosages known to those of skill in the art using pharmaceutically acceptable carrier, which are well known to those of skill in the art. More specifically, the dosage is dependent upon the specific usage of glutamine, and the form of administration. Preferably, the pharmaceutical carrier is altered based upon the intended use of the composition. Additionally, the carrier can alter/control the rate of absorption or *in vivo* release of the composition to the patient, organ, tissue, or cell. In other words, the carrier can be modified to enable prolonged release of glutamine at the site of treatment. The benefit of such release is that it enables longer term treatment at the site. Additionally, the release can be altered to achieve optimal pharmacokinetics and pharmacodynamics in order to optimize the treatment results.

GLN can improve outcomes from the problems enclosed above including, but not limited to, ischemia and reperfusion injury in the heart when given as a single dose. For example, a single dose of glutamine dipeptide (alanyl-glutamine) given to a rat 18 hours prior to myocardial injury can improve myocardial function following ischemia and reperfusion injury. This protection is due to preservation of metabolism and glutathione levels. A single dose of alanyl-glutamine functions via preservation of tissue metabolism (ATP content etc.) and preservation of tissue glutathione levels. For example, GLN can be given as a large IV bolus prior to surgery. The dosage range is preferably from 0.1 grams/kg up to 2 grams/kg of glutamine per dose. If needed, additional quantities of GLN can be administered post-operation to provide sustained protection.

The composition of the present invention can be used to administer alanyl-glutamine or other glutamine dipeptide to patients coming to the ER with heart attacks, patients coming for cardiac surgery, patients with chronic angina, or patients exhibiting symptoms of other problems as disclosed above. The benefit of the treatment is enormous as this a benign and relatively cheap amino acid. A further benefit is that, as stated above, GLN clears the body within a few hours, therefore there is no problem with regard to toxicity.

In a more specific disclosure of the treatment disclosed above, glutamine is protective against cellular and organ injury *in vitro* and *in vivo.* The data provided herein established that glutamine pre-treatment can directly protect cardiomyocytes against I/R induced myocardial cell death while enhancing return of contractile function. Additionally, glutamine can enhance expression of heat shock proteins 72 and 27, which are endogenous cytoprotective factors, in the hearts of both stressed and unstressed animals. GLN pretreatment can preserve myocardial function following I/R injury. The GLN-mediated conservation of myocardial function was found to be associated with improved markers of myocardial tissue metabolism including improved ATP/ADP ratio, total ATP content, and total creatine/phosphocreatine content following I/R injury. Further, GLN pretreatment led to enhanced substrate levels of glutamate (a key substrate in the ischemic myocardium) prior to I/R injury and enhanced tissue levels of glutamate, glutamine, and glucose following I/R injury. These tissues also demonstrated an enhanced reduced glutathione and total NAD (+)/NADH content indicating a reduction in tissue oxidant generation following I/R injury (improved redox state) and preservation of mitochondrial oxidative phosphorylation. This study implicated preservation of cellular metabolites in prevention of myocardial I/R injury. Finally, recent data indicates that low-dose oral GLN can delay the onset of ischemia in patients with known stable chronic angina.

Additionally, glutamine (GLN) protects against endotoxin-induced mortality. However, the effect of GLN following cecal-ligation and double- puncture (2CLP), a more clinically relevant model of human sepsis, is unknown. Further, increased Interleukin-18 (IL-18) levels appear to play a critical role in cytokine-induced organ failure and can be predictive of mortality in sepsis patients. Post-treatment with GLN attenuates pro-inflammatory (IL-18, IL-6, and TNF-α cytokine expression, enhance lung heat shock protein 72 (HSP 72) expression, and improves survival following 2CLP. Sprague-Dawley rats (250-300g) underwent 2CLP and one hour following surgery a single dose of GLN (0.75 g/Kg) (n=18) or a balanced salt solution (control) (n=17) was administered. Survival was monitored for five days. In a separate group of animals, blood was drawn at multiple time points for analysis of cytokine (IL-18, IL-6 TNF-α, and IL-10) expression. Lung tissue was harvested for analysis of HSP 72. GLN administration one hour following 2CLP significantly attenuated the expression of IL-18 at 12, 18, and 24 hours post-2CLP (p < 0.05). Expression of IL-6 and TNF-α was attenuated at 6 hours post-2CLP (p < 0.05). GLN enhanced lung HSP 72 expression versus control (p < 0.05) following 2CLP. Finally, GLN decreased the 5-day mortality rate from 78% to 42% (p <.04) following 2CLP. The data indicates that GLN can markedly improve survival from 2CLP. Potential mechanisms for this protection include attenuation of pro-inflammatory cytokine expression and enhanced lung HSP 72 expression. Further, this is the first description of IL-18 expression in the 2CLP model. These data indicate that a single dose of GLN given as post-treatment can attenuate the systemic inflammatory response syndrome (SIRS) and prevent lethality from polymicrobial sepsis.

The invention is further described in detail by reference to the following experimental examples. These examples are provided for the purpose of illustration only, and are not intended to be limiting unless otherwise specified. Thus, the invention should in no way be construed as being limited to the following examples, but rather, should be construed to encompass any and all variations which become evident as a result of the teaching provided herein.

### EXAMPLES

### Delivery of therapeutics (compounds):

The compound of the present invention is administered and dosed in accordance with good medical practice, taking into account the clinical condition of the individual patient, the site and method of administration, scheduling of administration, patient age, sex, body weight and other factors known to medical practitioners. The pharmaceutically "effective amount" for purposes herein is thus determined by such considerations as are known in the art. The amount must be effective to achieve improvement including but not limited to improved survival rate or more rapid recovery, or improvement or elimination of symptoms and other indicators as are selected as appropriate measures by those skilled in the art.

The compound of the present invention can be administered in various ways. It should be noted that it can be administered as the compound or as pharmaceutical acceptable salt and can be administered alone or as an active ingredient in combination with pharmaceutically acceptable carriers, diluents, adjuvants and vehicles. The compounds can be administered orally, subcutaneously or parenterally including intravenous, intraarterial, intramuscular, intraperitoneally, and intranasal administration as well as intrathecal and infusion techniques. Implants of the compounds are also useful. The patient being treated is a warm-blooded animal and, in particular, mammals including man. The pharmaceutically acceptable carriers, diluents, adjuvants and vehicles as well as implant carriers generally refer to inert, non-toxic solid or liquid fillers, diluents or encapsulating material not reacting with the active ingredients of the invention.

It is noted that humans are treated generally longer than the mice or other experimental animals exemplified herein which treatment has a length proportional to the length of the disease process and drug effectiveness. The doses may be single doses or multiple doses over a period of several days, but single doses are referred.

The doses may be single doses or multiple doses over a period of several days. The treatment generally has a length proportional to the length of the disease process and drug effectiveness and the patient species being treated.

When administering the compound of the present invention parenterally, it will generally be formulated in a unit dosage injectable form (solution, suspension, emulsion). The pharmaceutical formulations suitable for injection include sterile aqueous solutions or dispersions and sterile powders for reconstitution into sterile injectable solutions or dispersions. The carrier can be a solvent or dispersing medium containing, for example, water, ethanol, polyol (for example, glycerol, propylene glycol, liquid polyethylene glycol, and the like), suitable mixtures thereof, and vegetable oils.

Proper fluidity can be maintained, for example, by the use of a coating such as lecithin, by the maintenance of the required particle size in the case of dispersion and by the use of surfactants. Nonaqueous vehicles such a cottonseed oil, sesame oil, olive oil, soybean oil, corn oil, sunflower oil, or peanut oil and esters, such as isopropyl myristate, may also be used as solvent systems for compound compositions. Additionally, various additives which enhance the stability, sterility, and isotonicity of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. In many cases, it will be desirable to include isotonic agents, for example, sugars, sodium chloride, and the like. Prolonged absorption of the injectable pharmaceutical form can be brought about by the use of agents delaying absorption, for example, aluminum monostearate and gelatin. According to the present invention, however, any vehicle, diluent, or additive used would have to be compatible with the compounds.

Sterile injectable solutions can be prepared by incorporating the compounds utilized in practicing the present invention in the required amount of the appropriate solvent with various of the other ingredients, as desired.

A pharmacological formulation of the present invention can be administered to the patient in an injectable formulation containing any compatible carrier, such as various vehicle, adjuvants, additives, and diluents; or the compounds utilized in the present invention can be administered parenterally to the patient in the form of slow-release subcutaneous implants or targeted delivery systems such as monoclonal antibodies, vectored delivery, iontophoretic, polymer matrices, liposomes, and microspheres. Examples of patents disclosing delivery systems useful in the present invention include: 5,225,182; 5,169,383; 5,167,616; 4,959,217; 4,925,678; 4,487,603; 4,486,194; 4,447,233; 4,447,224; 4,439,196; and 4,475,196. Many other such implants, delivery systems, and modules are well known to those skilled in the art.

Known techniques that deliverthe pharmacological formulation of the compound utilized in the present invention intravenously and retain the biological activity are preferred.

The compound of the present invention can be administered initially by intravenous injection to bring blood levels to a suitable level. The patient's levels are then maintained by an oral dosage form, although other forms of administration, dependent upon the patient's condition and as indicated above, can be used. The quantity to be administered will vary for the patient being treated and will vary from about 100 ng/kg of body weight to 100 mg/kg of body weight per day and preferably will be from 10 mg/kg to 10 mg/kg per day.

### Example 1:

Myocardial ischemia/reperfusion (I/R) injury is an unavoidable consequence of surgical procedures requiring cardiopulmonary bypass. Due to lack of a successful therapeutic regimen, pre-operative prophylaxis against myocardial I/R injury during procedures requiring cardiopulmonary bypass is not a clinically routine procedure. Preliminary data from the research and other laboratories indicates Glutamine (GLN), a conditionally essential amino acid, is protective against cellular and organ injury *in vitro* and *in vivo.* The data shows that glutamine pre-treatment can directly protect cardiomyocytes against I/R induced myocardial cell death while enhancing return of contractile function.

Additionally, glutamine can enhance expression of heat shock protein 72 and 27, which are endogenous cytoprotective factors, in the hearts of both stressed and unstressed animals. Subsequent data from the laboratory indicates that GLN pretreatment to the intact rat can preserve myocardial function following I/R injury in a working heart model. This GLN-mediated conservation of myocardial function was found to be associated with improved markers of myocardial tissue metabolism including improved ATP/ADP ratio, total ATP content, and total creatine/phosphocreatine content following I/R injury. Further, GLN pretreatment led to enhanced substrate levels of glutamate (a key substrate in the ischemic myocardium) prior to I/R injury and enhanced tissue levels of glutamate, glutamine, and glucose following I/R injury. The tissues also demonstrated an enhanced reduced glutathione and total NAD (+)/NADH content indicating a reduction in tissue oxidant generation following I/R injury (improved redox state) and preservation of mitochondrial oxidative phosphorylation. Data from another laboratory has corroborated these findings and demonstrated that GLN administration via the perfusate in a rat working heart can improve outcome from I/R injury. The study implicated preservation of cellular metabolites in prevention of myocardial I/R injury. Finally, recent data indicates that low-dose oral GLN can delay the onset of ischemia in patients with known stable chronic angina. Therefore, pre-operative GLN therapy can attenuate myocardial injury as measured by coronary effluent and plasma troponin and creatinine phosophokinase release.

**The effect of GLN on myocardial tissue injury.** The preoperative administration of GLN to patients undergoing CABG surgery decreases myocardial cell injury following cardipulmonary bypass. Plasma can be collected pre-operatively, at initiation of cardiopulmonary bypass, and post-operatively and analyzed for troponin and CK-MB. Additionally, coronary effluent can be collected at various points during cardiopulmonary bypass and anlayzed for troponin.

**The effect of GLN on human atrial tissue heat shock protein content.** The preoperative administration of GLN to patients undergoing CABG surgery enhances myocardial heat shock proteins 72 and 27 content at initation and conclusion of cardiopulmonary bypass. Right atrial appendage tissue can be harvested from patients undergoing CABG surgery pre- and post- cardiopulmonary bypass and analyzed for heat shock protein 72 and 27 content (Western analysis).

**The effect of GLN on enhancement of measures of tissue metabolism and redox state in human atrial tissue.** The preoperative administration of GLN to patients undergoing CABG surgery improves measurements of myocardial tissue metabolism at initation and discontinuation of cardiopulmonary bypass. Right atrial appendage tissue can be harvested from patients prior to initiation of coronary artey bypass and analyzed for tissue metabolism (magnetic resonance spectroscopy (MRS)). These measurements include tissue ATP, ATP/ADP ratio, lactate, glutamine, gluatamte, and glucose. Tissue redox state and preservation of oxidative phosphorylation can be analyzed via mycardial tissue content of reduced glutathione and total NAD/NADH content (MRS).

**The effect of GLN on post-operative cardiac function and overall patient outcomes.** The preoperative administration of GLN to patients undergoing CABG surgery can (i) improve post-operative cardiac function, (ii) decrease need for post-operative vasopressor support, and (iii)shorten length of post-operative mechanichal ventilation, ICU, and hospital length of stay. Data can be collected on cardiac function pre-opeartively, at initiation and termination of cardiopulmonary bypass, and post-operatively (via cardiac index). Requirement for vasopressor therapy post-operatively can be assesed for both dose and length of time patient requires vasopressor treatment. Additionally, duration of post-operative ventilation, length of ICU admission, and total length of hospital stay can be recorded.

Insight gained from the above sheds light on the potential for GLN pretreatment to protect against myocardial I/R injury following cardiopulmonary bypass for CABG. Further, the studies provide further insight into the biochemical mechanisms by which GLN can protect against I/R injury in the human myocardium.

Preliminary data in human patients indicates that the administration of low dose oral GLN (80 mg/kg) can significantly delay the time to onset of ischemia in patients with chronic stable angina. Specifically, the randomized, blinded, placebo controlled crossover trial examined the effect of GLN given to 17 patients with known chronic stable angina. Patients were given GLN or placebo 40 minutes prior to an exercise stress test and the time until onset of 1 mm of ST depression was recorded. Patients were then crossed over following a one-week interval. GLN administration led to a significant delay in the onset of ST depression (p < 0.05).

GLN has previously been administered to critically ill humans in a variety of clinical settings without any evidence of toxicity. The only exception to this finding has been in patients with significant renal and/or liver failure. The patients were observed to have a rise in their blood urea nitrogen and ammonia levels that were not reported to have serious physiologic consequences and were reversible with discontinuation of the therapy.

In addition to patients undergoing CABG procedures, other groups of patients can benefit from potential GLN-mediated protection against myocardial I/R injury. In patients at risk for cardiac ischemia (such as in patients with coronary artery disease and/or chronic unstable angina) GLN administration can reduce myocardial injury and improve cardiac performance following myocardial ischemia or infarction. Further, GLN can also be used to protect against ischemic injury in hearts and other organs harvested for organ transplantation.

Glutamine can improve clinical outcomes of critical illness; the mechanism of this is unknown Presently glutamine is given as a nutritional supplement to parenteral nutrition or enteral feeding. A single large dose of glutamine given as a "drug" can markedly improve outcomes of sepsis, acute respiratory distress syndrome, and pro-inflammatory states by correcting defective heat shock protein 70 expression, attenuating pro-inflammatory cytokine release, and correcting sepsis induced defects in metabolism.

Giving glutamine at 0.75 g/kg as a single dose (of alanyl-glutamine) can enhance heat shock protein 70 expression, attenuate pro-inflammatory cytokine expression (TNF-alpha, IL-6, IL-18 exc.), and improve tissue metabolism (improved tissue ATP levels, decreased lactate, and preserved NAD + levels) leading to decreased mortality and morbidity following sepsis and acute respiratory distress syndrome. This can have significant clinical implications for improving outcomes from sepsis, lung injury, trauma, and other inflammatory disorders (such as arthritis, inflammatory bowel disease, and multiple sclerosis)

Alanyl-glutamine was administered and it was determined that administration following onset of sepsis in the clinically relevant cecal-ligation and puncture model of sepsis in the rat that alanyl-glutamine, given as a single large dose, can restore normal heat shock protein expression to the lung, attenuate inflammatory cytokine release, and improve otherwise defective cellular metabolism in the lung.

Large single or multiple doses of the amino acid dipeptide can be used to significantly improve outcomes from severe infections, acute respiratory distress syndrome, and other severe injuries like trauma and severe bums. The dose can be given upon admission to the emergency room or ICU to prevent morbidity and mortality. Also, the amino acid can improve outcomes from other inflammatory diseases such as inflammatory bowel disease (Crohns disease, ulcerative colitis), arthritis, multiple sclerosis, and/or lupus.

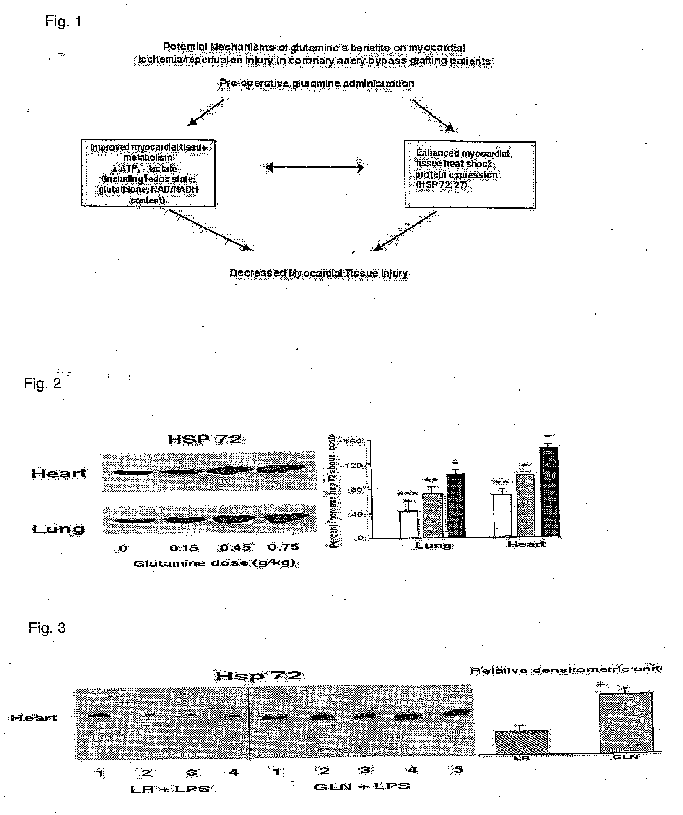

**GLN enhances heat shock protein expression in the unstressed and stressed myocardium.** In the initial studies the effect of GLN on myocardial heat shock protein expression in the stressed and unstressed intact rat was examined. The first set of experiments examined the effect of a range of GLN doses on heat shock protein expression in the unstressed rat heart. As shown below in Figure 1, GLN significantly enhanced HSP 72 expression at all doses tested: The increase was also observed for HSP 27 (another of the protective heat shock proteins) wherein the induction occurred as early as 4-6 hours and persisted for up to 48-72 hours. In a separate set of experiments a group of rats were treated with 0.75 g/kg of GLN or a LR control solution and then concurrently given 5mg/kg of Escherichia Coli endotoxin (LPS). Six hours following GLN/LPS administration animals were sacrificed and heart tissue was harvested for analysis of HSP expression. GLN markedly enhanced both HSP 72 and 27 expression as measured by western blot analysis (Figure 2).

More specifically, Figure 2 shows the dose response effect of GLN on HSP 72 expression in heart and lung tissue in unstressed rats. Rats were treated with varying concentrations of GLN given over 12-15 minutes. Tissues were then harvested four hours post-GLN administration, and HSP 72 expressions were analyzed by Western blot. The image shown is representative of those obtained in four different experiments. The graph represents a percent increase in HSP 72 over control. All GLN doses (0.15 g/kg (open bars), 0.45 g/kg (gray bars) 0.75 g/kg (black bars)) significantly increased HSP 72 concentrations (measured by densitometry) over control. (* p < .001, ** p < .01, *** p< .05 compared to control by ANOVA.)

Figure 3 shows the effect of GLN on HSP 72 expression in hearts of LPS-injured rats. Rats were given LPS (5 mg/kg) concomitantly with GLN (n = 5) + fluid resuscitation or LR resuscitation alone (n = 4) via the tail vein. Tissues were harvested six hours post-LPS injury and analyzed for HSP 72 expression via Western blot. Each lane represents protein extracts from a separate animal. Relative densitometry are expressed as means +/- SE. *- p < 0.0005, compared with LR resuscitation alone assessed by Students t test.

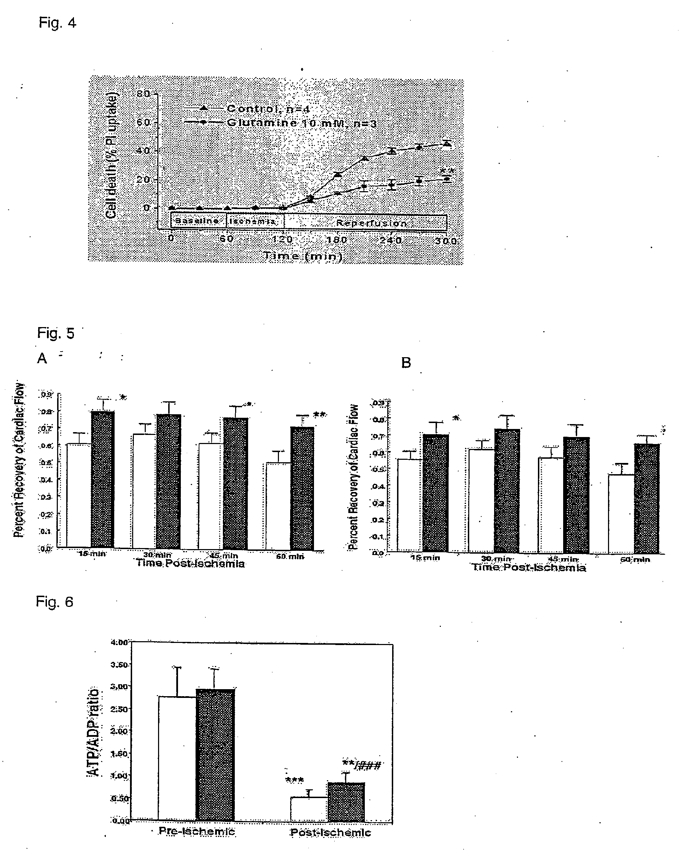

**GLN protects cardiomyocytes against I/R injury and enhances heat shock protein 72 expression**. In further preliminary studies, whether GLN can protect isolated cardiomyocytes against I/R injury was tested. Spontaneously contracting cardiomyocytes were studied. Glutamine pretreatment was achieved by allowing cardiomyocytes to equilibrate in BSS with 10 mM or 0 mM GLN added for one hour prior to subsequent ischemia/reperfusion. GLN was also present throughout ischemia and reperfusion period in the GLN treated cells. Significant decreases in cell death in the glutamine treated cells (22 %) versus control cells (48%) (p < .0005) were observed (Figure 4). GLN also significantly enhanced return of contractile function following I/R injury (p < .05). GLN treated cells further demonstrated a significant increase in HSP 72 content as measured by western blot versus non-preconditioned cells (cells not receiving GLN).

Figure 4 shows the effect of Glutamine (GLN) on cell death from ischemia/reperfusion. Cardiomyocytes were allowed to equilibrate at baseline normoxic conditions for 60 minutes. During this period, cells were exposed to a balanced salt solution containing 10 mM of GLN or balanced salt solution alone (control). (** - p < .0005 relative to control)

**Glutamine pretreatment enhances recovery of cardiac and coronary flow in rat working heart following I/R injury**. To examine the effect of remote GLN pre-conditioning on recovery of cardiac function in an intact animal, a rat working heart model was utilized. Rats were injected with 0.75 g/kg of an alanine-glutamine solution (equivalent to .52 g/kg glutamine) (n=10) or a control Lactated Ringer's (LR) solution (n=10) 18 hours prior to heart harvest and exposure to an I/R injury via a working heart apparatus. Pre-load values were examined for both (8 and 12 mmHg), GLN preconditioning significantly improved recovery of cardiac flow following reperfusion (Figure 5). GLN preconditioning also significantly enhanced percent recovery of coronary flow at 60 minutes following reperfusion (p < .01). No visible toxicity (abnormal behavior, change in mental status, decreased feeding) was noted from GLN or control injections.

Figure 5 shows the effect of glutamine (GLN) pretreatment on cardiac indices following ischemia/reperfusion injury in rat working heart model. Rats were injected IP with 0.52 g/kg GLN (as alanine-glutamine) or a LR control solution 18 hours prior to heart harvest and experimental ischemia/reperfusion injury. Figure 5A shows the percent recovery of cardiac flow at a left atrial pressure of 8 mm Hg. Figure 5B shows the percent recovery of cardiac flow at a left atrial pressure of 12 mm Hg. *- ≤ 0.05, ** - p ≤ 0.01 relative to control.

**Glutamine pretreatment preserves myocardial ATP/ADP ratio and total ATP/ creatine/phosphocreatine content following myocardial I/R injury in the rat**. Rat working heart model myocardial tissue was collected from the rats described in Figure 5 above following the 60-minute reperfusion time. Control animals treated with GLN or LR also had myocardial tissue harvested at 18 hours, but this tissue was not exposed to I/R injury (Pre-ischemic tissue) and was immediately frozen in liquid nitrogen for analysis. The tissue was analyzed for ATP, ADP, and ATP/ADP ratio via ³¹P- magnetic resonance spectroscopy (MRS) and for creatine/phosphocreatine content via ¹H- magnetic resonance spectroscopy (MRS). Both groups of animals had a significant decrease in ATP/ADP ratio and total ATP following I/R injury. However, pre-treatment with GLN 18 hours prior to myocardial I/R injury led to an increased preservation of ATP/ADP ratio following I/R injury (figure 6). Total ATP content was also improved (p < 0.001 versus control via students t-test).

ATP depletion is a major contributor to myocardial dysfunction and myocardial cell death following I/R injury. GLN pretreatment 18 hours prior to myocardial I/R injury markedly preserves myocardial ATP content, which can be a vital mechanism by which GLN enhances myocardial function following I/R injury. No statistically significant decreases in creatine/phosphocreatine content were observed in the animals undergoing I/R injury. However, pre-treatment with GLN 18 hours prior to myocardial I/R injury led to enhanced myocardial tissue creatine/phosphocreatine content following I/R injury. The failing myocardium was characterized by reduction of phosphocreatine (PCr) and free creatine contents. Rats with a significant decrease in myocardial phosphocreatine cannot survive significant myocardial I/R injury. GLN can preserve the creatine/phosphocreatine content of the ischemic - heart, which is vital to the maintenance of ATP homeostasis and helps to explain the improved post-reperfusion myocardial function in these rat hearts.

Figure 6 shows the effect of myocardial ischemia/reperfusion injury on myocardial tissue ATP/ADP ratio in rat working heart model. Rats were injected IP with a LR control solution (n =10) (open bars) or 0.52 g/kg GLN (as alanine-glutamine) (n=10) (solid bars) 18 hours prior to heart harvest and experimental ischemia/reperfusion injury. Post-ischemic tissue was harvested 60 minutes following reperfusion. ATP/ADP ratio was analyzed via ³¹P- MRS. (***- p < 0.001, ** p < 0.01 versus pre-ischemic values, ###- p < 0.001 versus post-ischemic LR control tissue via ANOVA.)

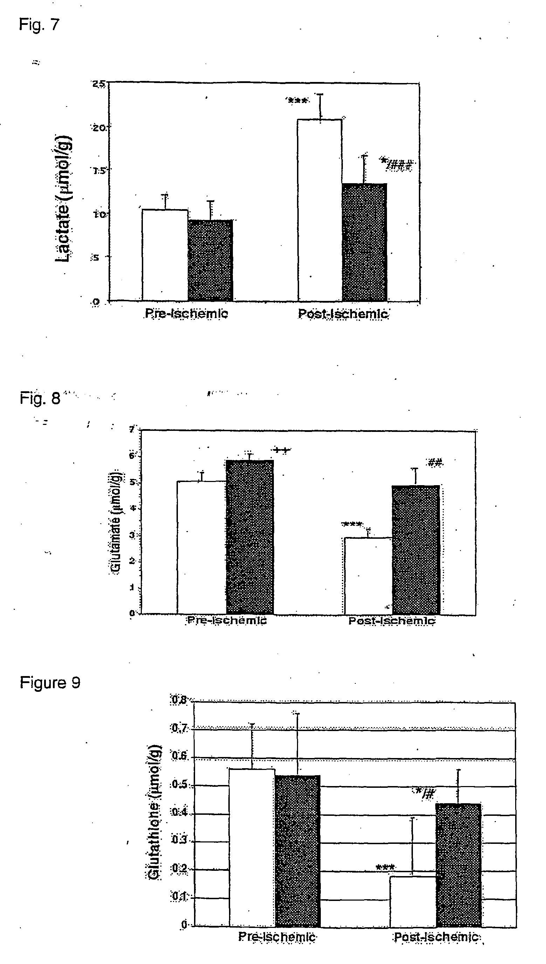

**Glutamine pretreatment decreases myocardial lactate accumulation following myocardial I/R injury in the rat**. Utilizing the aforementioned rat working heart model, myocardial tissue was collected from the rats described in Figure 5 above following the 60-minute reperfusion time. Control animals treated with GLN or LR also had myocardial tissue harvested at 18 hours, but the tissue was not exposed to I/R injury (Pre-ischemic) and was immediately frozen in liquid nitrogen for analysis. The tissue was analyzed for lactate content via ¹H-MRS. Both groups of animals exposed to I/R injury had a significant increase in myocardial tissue lactate over control animals. However, pre-treatment with GLN 18 hours prior to myocardial I/R injury led to a significant decrease in accumulation of myocardial lactate following I/R injury (Figure 7). Specifically, GLN pre-treatment reduced the increase in tissue lactate by > 55% versus control animals. This indicates that GLN can decrease myocardial tissue acidosis following I/R injury. The decrease is due to GLN induced preservation of myocardial tissue metabolism, particularly preservation of proper TCA cycle function. The decrease can occur via GLN providing substrate for metabolism in the TCA cycle (via conversion to glutamate) or via GLN induced preservation of TCA cycle enzyme function (via HSP induction).

Figure 7 shows the effect of myocardial ischemia/reperfusion injury on myocardial tissue lactate in rat working heart model. Rats were injected IP with a LR control solution (n =10) (open bars) or 0.52 g/kg GLN (as alanine-glutamine) (n=10) (solid bars) 18 hours prior to heart harvest and experimental ischemia/reperfusion injury. Post-ischemic tissue was harvested 60 minutes following reperfusion. Lactate was analyzed via ¹H- MRS. (***- p < 0.001, * p < 0.05 versus pre-ischemic values, ###- p < 0.001 versus post-ischemic LR control tissue via ANOVA.)

**Glutamine pretreatment enhances myocardial tissue content (glutamine, glutamate, and glucose) content following myocardial I/R injury in the rat.** Utilizing the aforementioned rat working heart model, myocardial tissue was collected from the rats described in Figure 5 above following the 60-minute reperfusion time. Control animals treated with GLN or LR (LR) also had myocardial tissue harvested at 18 hours (Pre-ischemic), but the tissue was not exposed to I/R injury and was immediately frozen in liquid nitrogen for analysis.. The tissue was analyzed for glutamine, glutamate, and glucose content via ¹H- magnetic resonance spectroscopy (MRS). Both GLN and LR control animals exposed to I/R injury had decreases in GLN and "glucose content following I/R injury. However, GLN treated animals demonstrated no decrease in post-ischemic glutamate levels from pre-ischemic levels. Glutamate is a vital tissue substrate following myocardial injury and it is beneficial that a single dose of GLN 18 hours prior to I/R injury can preserve tissue levels of this key substrate following injury. Further, pre-treatment with GLN 18 hours prior to myocardial I/R injury led to enhanced myocardial tissue content of glutamine and glucose following I/R injury versus LR control animals (Figure 8). The data indicates that GLN pre-treatment can enhance the myocardial tissue content of a number of the key substrates utilized following I/R injury. This key finding confirms that it is possible to enhance myocardial tissue substrate content (particularly glutamate) with a (pre-operative) remote GLN pre-treatment dose.

More specifically, Figure 8 shows the effect of myocardial ischemia/reperfusion injury on myocardial tissue substrate (glutamate, glutamine, and glucose) in rat working heart model. Rats were injected IP with a LR control solution (n =10) (open bars) or 0.52 g/kg GLN (as alanine-glutamine) (n=10) (solid bars) 18 hours prior to heart harvest and experimental ischemia/reperfusion injury. Post-ischemic tissue was harvested 60 minutes following reperfusion. Substrates were analyzed. via ¹H- MRS. (++ - p < 0.01 versus pre-ischemic LR control, ***- p < 0.001, * p < 0.05 versus pre-ischemic values, ##- p < 0.01, ###- p < 0.001 versus post-ischemic LR control tissue via ANOVA.)

**Glutamine pretreatment enhances myocardial tissue reduced glutathione content following myocardial I/R injury in the rat. Utilizing the** aforementioned rat working heart model myocardial tissue was collected from the rats described in Figure 5 above following the 60-minute reperfusion time. Control animals treated with GLN or LR (LR) also had myocardial tissue harvested at 18 hours (Pre-ischemic), but the tissue was not exposed to I/R injury and was immediately frozen in liquid nitrogen for analysis. The tissue was analyzed for myocardial tissue reduced glutathione content via ¹H- magnetic resonance spectroscopy (MRS). Both GLN and LR control animal undergoing I/R injury showed reductions in reduced GSH content, however the animals treated with GLN showed a minimal decrease versus that seen in the LR control animals. Specifically, pre-treatment with GLN 18 hours prior to myocardial I/R injury led to a marked enhancement myocardial tissue reduced glutathione content following I/R injury (Figure 9).

GSH depletion can exacerbate cardiac dysfunction inflicted by myocardial I/R injury. Part of the etiology can involve impaired myocardial antioxidant defenses. Further, GLN has been shown to preserve GSH content and decrease injury in the myocardium following injury with doxorubicin. Finally, GLN is known to be a rate-limiting precursor for glutathione synthesis and thus the enhanced glutathione content observed following myocardial I/R injury can reflect enhanced precursor availability for glutathione production.

Figure 9 shows the effect of myocardial ischemia/reperfusion injury on myocardial tissue reduced glutathione content in rat working heart model. Rats were injected IP with a LR control solution (n =10) (open bars) or 0.52 g/kg GLN (as alanine-glutamine) (n=10) (solid bars) 18 hours prior to heart harvest and experimental ischemia/reperfusion injury. Post-ischemic tissue was harvested 60 minutes following reperfusion. Lactate was analyzed via ¹H- MRS. (***- p < 0.001, * p < 0.05 versus pre-ischemic values, #- p < 0.05 versus post-ischemic LR control tissue via ANOVA.)

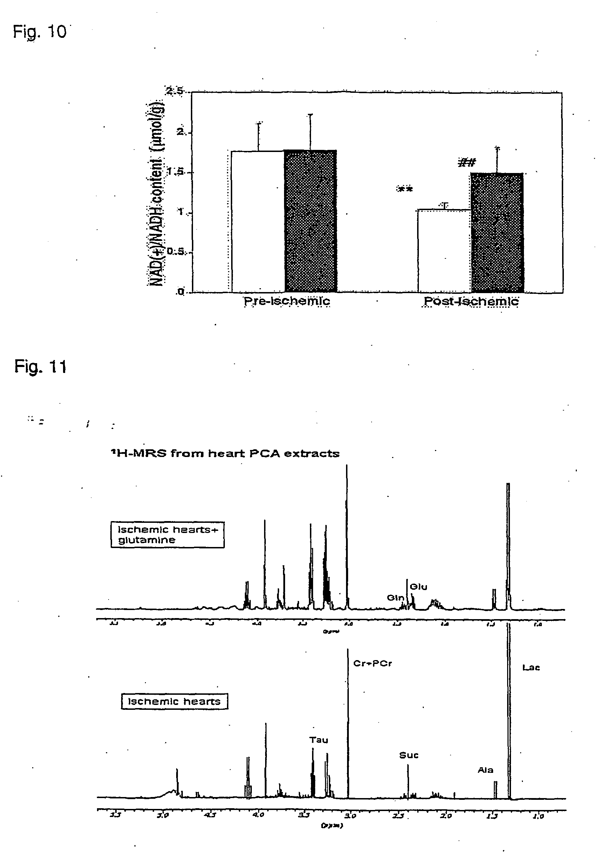

**Glutamine pretreatment preserves myocardial NAD (+)/NADH content following myocardial UR injury in the rat.** Utilizing the aforementioned rat working heart model myocardial tissue was collected from the rats described in Figure 5 above following the 60-minute reperfusion time. Control animals treated with GLN or LR (LR) also had myocardial tissue harvested at 18 hours, but the tissue was not exposed to I/R injury (Pre-ischemic) and was immediately frozen in liquid nitrogen for analysis. The tissue was analyzed for NAD (+)/NADH content via ³¹P- magnetic resonance spectroscopy (MRS). Only the animals in the LR control group exposed to I/R injury demonstrated a decrease in total NAD (+)/NADH content versus pre-ischemic conditions. No change was seen in post-ischemic NAD (+)/NADH content (versus pre-ischemic content) in animals treated with GLN. Specifically, treatment with GLN 18 hours prior to myocardial I/R injury led to a significant preservation of NAD (+)/NADH content following I/R injury (Figure 10).

Preservation of NADH content is vital to the myocardium's ability to withstand the inevitable production of reactive oxygen species following I/R injury. Previously, substrate enhancement with pyruvate and other amino acids has been shown to preserve NAD (+)/NADH content and protect against myocardial contractile dysfunction and myocardial tissue injury. GLN is acting in a similar fashion (via substrate enhancement) to preserve NADH content and decrease myocardial tissue injury and and improve myocardial contractility. Further, increased NAD (+)/NADH content is a marker for enhanced mitochondrial oxidative phosphorylation. The increased content explains the previously described improvement in ATP/ADP ratio following GLN pre-treatment.

Figure 10 shows the effect of myocardial ischemia/reperfusion injury on myocardial tissue NAD(+)/NADH content in rat working heart model. Rats were injected IP with a LR control solution (n =10) (open bars) or 0.52 g/kg GLN (as alanine-glutamine) (n=10) (solid bars) 18 hours prior to heart harvest and experimental ischemia/reperfusion injury. Post-ischemic tissue was harvested 60 minutes following reperfusion. NAD(+)/NADH was analyzed via ¹H- MRS. (**- p < 0.01 versus pre-ischemic values, ##- p < 0.01 versus post-ischemic LR control tissue via ANOVA.)

**NMRIMRS spectra of rat hearts exposed to myocardial I/R injury.** For specific examples of ¹H-MRS see Figure 11 below. Figure 11 shows ¹H-MRS spectra from rat myocardial tissue exposed to ischemia and reperfusion injury in rat working heart model. Rats were injected IP with a LR control solution or 0.52 g/kg GLN 18 hours prior to heart harvest and experimental ischemia/reperfusion injury. Post-ischemic tissue was harvested 60 minutes following reperfusion. (GLN=glutamine, GLU=glutamate, Tau=taurine, CR+PCr=creatine+phosphocreatine, Suc=succinate, Ala=alanine, Lac=lactate).

**Treatment and randomization procedure.** Patients randomized to the GLN group (treatment group) received oral L-GLN 50grams (25 grams orally, 2 times a day) for two days prior to their operative procedure and can also take 25 grams of GLN on the morning of surgery. Patients randomized to the placebo received an oral placebo mixture (2 time a day) for two days prior to their operative procedure and also received the mixture on the morning of surgery. Placebo consisted of a maltodextran powder (25 grams per dose). Patients randomized to the iso-nitrogenous control group received a orally administered control mixture (2 time a day) for two days prior to their operative procedure and also received the mixture on the morning of surgery. The iso-nitrogenous control treatment consisted of a mixture of amino acids (not containing glutamine or glutamate) iso-nitrogenous to 25 grams of GLN.

During admission patients also received a standardized diet that can be controlled for protein content by the GCRC dieticians to administer approximately 20% of patients daily caloric requirement as protein and 20% as fat, with the remaining 60% administered as carbohydrate calories. The diet was done so as to control for pre-operative dietary intake.

The selected dose of GLN is based on the maximum dose that has been previously been administered safely in humans. The dose has been tolerated well tolerated without adverse side effects, even in critically ill patients (26-30). Blood was also drawn to assess GLN levels throughout the study. The first blood draw occurred at admission (< then 3 cc's of blood). An additional GLN level was obtained one hour after the first dose of GLN/placebo/iso-nitrogenous control, a GLN level was obtained prior to induction of anesthesia, and a final GLN level at 24 hours post- CABG procedure.

### Methods:

### Determine the effect of GLN on myocardial tissue injury

**Evaluation of myocardial tissue injury.** The following laboratory test can be drawn two days prior to CABG procedure; Creatine phoshphokinase (CPK) (with MB isoforms) and troponin analysis.

Pre-operatively, after induction of anesthesia, but before sternal opening, approximately 5 cc's of blood was drawn from patients for the following: CPK-MB, and troponin analysis. At initiation of the bypass procedure and two minutes after the release of the aortic cross clamp coronary effluent was evaluated for troponin and CPK-MB levels. Post-operatively, prior to transfer to ICU, approximately 5 cc's of blood was drawn for CPK-MB, and troponin. At 6 and 24 hours post-operatively, again a blood sample was taken for CPK-MB and troponin (approximately 5 cc's of blood).

Pre-operative GLN pre-treatment can significantly attenuate myocardial injury as measured by plasma and coronary effluent troponin and CPK-MB measurement. Pre-clinical data in both cellular and whole animal models shows that GLN pre-treatment appears to protect against myocardial I/R injury and this protection was also observed in human subjects undergoing CABG surgery.

**Statistical Analysis.** Normality of distribution of data was checked by visual inspection of Q-Q plots. A log transformation was applied when necessary and checked again for normality. Differences in markers of cardiac injury was analyzed via ANOVA for repeated measures; if a significant overall difference is observed between the groups is found, differences at individual time points was analyzed by means of the students t test. A p value <0.05 was considered significant. The SPSS software package (version 10.07, SPSS Inc., Chicago IL) was used for all statistical analyses.

### Determine the effect of GLN on human atrial tissue heat shock protein content.

**Right Atrial Tissue Harvest:** During coronary artery bypass procedure, at the time of atrial cannulation, patients had a portion of the right atrial appendage removed by the investigators and immediately frozen in liquid nitrogen. Additionally, at the conclusion of cardiopulmonary bypass additional right atrial appendage tissue was removed from around the cannulation site and immediately frozen in liquid ni**trogen. This tissue can be evaluated for heat shock protein.**

**Western Blot Analysis:** Harvested atrial tissues can be analyzed for heat shock protein 72 and 27 content via Western immunoblotting, via the technique previously described. HSP 72 content was analyzed via densitometry.

GLN therapy enhances HSP 72 and 27 levels in the right atrial tissue if patients undergoing CABG. Pre-stress changes in HSP levels have been observed following GLN administration in a number of previous cellular and animal models. However, GLN has its most profound effect on tissue HSP expression following injury. However, given that the patients in the present study all have some degree of coronary ischemia, the chronic I/R injury that occurs in many of these patients can lead to GLN enhancement of HSP levels in atrial tissue harvested pre-cardiopulmonary bypass as well.

**Statistical Analysis:** Normality of distribution of data was checked by visual inspection of Q-Q plots. A log transformation was applied when necessary and checked again for normality. Differences in right atrial tissue heat shock protein expression via ANOVA for repeated measures was analyzed; if a significant overall difference was observed between the groups is found, differences at individual time points was analyzed by means of the students t test. A p value < 0.05 was considered significant. The SPSS software package (version 10.07, SPSS Inc., Chicago IL) was used for all statistical analyses.

**Determine the effect of GLN on cell metabolism in human atrial tissue.** Right atrial appendage tissue were harvested from patients prior to initiation of coronary artery bypass and immediately following discontinuation of cardiopulmonary bypass to be analyzed for cell metabolism by ¹H- and ³¹P -magnetic resonance spectroscopy (MRS). ³¹P-MRS provided information about atrial tissue concentrations of high-energy phosphates (ATP, ADP, phosphocreatine, NAD(+), ATP/ADP ration). The following water-soluble metabolites were quantified from ¹H-MRS of atrial tissue extracts: alanine, aspartate, creatine+phosphocreatine, glucose, glutamate, glutamine, glutathione, glycerophosphocholine, lactate, phosphocholine, succinate, taurine, and valine+leucine+isoleucine. The following lipid metabolites were quantified from ¹H-MRS spectra of lipid extracts: cholesterol, cholines, glycerol phosphate, mono-unsaturated fatty acids (MUFA), phosphatidylcholine, phosphatidylethanolamine poly-unsaturated fatty acids (PUFA), total fatty acids, and triacylglycerides.

**Right Atrial Tissue Harvest:** During the coronary artery bypass procedure, at initiation of cardiopulmonary bypass, patients had a portion of the right atrial appendage removed by the investigators and immediately frozen in liquid nitrogen. Right atrial tissue from around the cannulation site was also harvested following discontinuation of cardiopulmonary bypass and immediately frozen in liquid nitrogen. Tissue was subsequently divided for analysis of cellular metabolism and heat shock protein.

**Dual PCA/ lipid extraction.** To perform high-resolution magnetic resonance spectroscopy (MRS) on atrial tissues, the collected frozen samples was extracted using a dual perchloric acid (PCA)/ lipid extraction procedure as described in detail by Serkova et al. Snap frozen hearts tissue were weighed, homogenized in a mortar grinder in the presence of liquid nitrogen, and extracted with 4 ml ice-cold PCA (12%). For each extract, 0.3 to 0.5 g atrial tissue was needed. The samples were centrifuged, the aqueous phase removed, neutralized using KOH, and centrifuged once again. The lipid fraction was extracted from the pellets remaining after the aqueous extraction step. The pellets were dissolved in 4 ml ice-cold water and the solution was neutralized. Both, the aqueous extract and the redissolved pellet, were lyophilized overnight. The lyophilisates of the aqueous extracts were redissolved in 0.45 ml deuterium oxide (D₂O) and adjusted to pH 7 using deuterium chloride (DCI) and deuteroxide (NaOD). The lipid fraction was extracted from the lyophilisates of the redissolved pellet by addition of 1 ml deuterated chloroform/ methanol mixture (CDCl₃/CD₃OD; 2:1 v/v). After centrifugation, MRS was analyzed the supernatants.

**MRS evaluation on right atrial tissue extracts.** MRS experiments based on ¹H- and ³¹P-nuclear magnetic resonance (NMR) spectroscopy were carried out as described previously (Serkova et al., (44)). All one-dimensional MR spectra of tissue PCA aqueous and lipid extracts were recorded on a Bruker 500 MHz Avance spectrometer and processed using WINNMR software (Bruker Biospec Inc., Fremont, CA). A 5 mm-TXI (inverse triple resonance) probe were used for all experiments. For proton MRS, the operating frequency can be 500 MHz, and a standard pre-saturation pulse program were used for water suppression. The other parameters were as follows: 40 accumulations; 90° pulse angle; 0 dB power level; 7.35 µs pulse width; 10 ppm spectral width; and 12.85 second repetition time. Trimethylsilyl propionic-2,2,3,3,-d₄ acid (TSP, 0.6 mmol/l for PCA extracts and 1.2 mmol/l for lipids) were used as external standards for the quantification of metabolites based on ¹H-MR spectra. ¹H chemical shifts of spectra were referenced to TSP at 0 ppm. For ³¹P-MRS analysis of PCA extracts, 100 mmol/I EDTA was added for complexation of divalent ions resulting in significantly narrower line width of ³¹P peaks. The pH was adjusted to 7 using KOH and HCl. The following NMR parameters with a composite pulse decoupling (CPD) program were used: 202.3 MHz operating ³¹P frequency; 800 accumulations; 90° pulse angle; 12 dB power level for ³¹P channel; 9 µs pulse width; 35 ppm spectral width; and 2.0 second repetition time. The chemical shift of phosphocreatine at -2.33 ppm was used as shift reference. The absolute concentrations of phosphocreatine calculated from ¹H-MRS were used for metabolite quantification of ³¹P-MR spectra.

**Anticipated Results:** GLN can enhance the cellular content of ATP, improved ratio of ATP/ADP, and enhanced content of NAD(+)/NADH, and creatine/phosphocreatine. Further GLN decreased myocardial tissue lactate, and improved myocardial tissue levels of glutamate, glutamine, and glucose. Finally, enhance levels of myocardial tissue glutathione.

**Statistical Analysis:** Normality of distribution of data was checked by visual inspection of Q-Q plots. A log transformation was applied when necessary and checked again for normality. Differences in markers of right atrial tissue metabolism via ANOVA for repeated measures were analyzed; if a significant overall difference was observed between the groups is found, differences at individual time points were analyzed by means of the students t test. A p value < 0.05 was considered significant. The SPSS software package (version 10.07, SPSS Inc., Chicago IL) was used for all statistical analyses.

### Determine the effect of GLN on post-operative cardiac function and overall patient outcomes.

**Evaluation of pre- and post-operative cardiac function.** In order to assess pre-operative cardiac function a continuous cardiac index/output swan ganz catheter was placed at induction of anesthesia. A cardiac output (CO), cardiac index (CI), and systemic vascular resistance (SVR) were recorded. Following discontinuation of cardiopulmonary bypass, immediately prior to sternal closure, cardiac parameters (CO, CI, and SVR) were recorded. At 6 and 24 hours following CABG procedure cardiac parameters (CO, CI, SVR) were assessed. Other data recorded can include the number of coronary vessels grafted and particular location of grafts (i.e. left main, right coronary, exedra.), length of cardiopulmonary bypass, length of aortic cross clamp, and red blood cell transfusion.

Additionally, the incidence of pre-operative cardiac arrhythmias (specifically: atrial fibrillation, atrial flutter, pre-mature ventricular complexes, ventricular tachycardia/fibrillation, and need for pacemaker/defibrillator, need for anti-arrhythmic therapy) was analyzed. The occurrence of arrhythmias post-operatively, as well as the need for pacing and or anti-arrhythmic therapy, were recorded.

**Evaluation of need for vasopressor therapy.** At initiation of anesthesia any requirement for vasopressor therapy and its dose were recorded. (Vasopressor therapy includes the use of any of the following medications: epinephrine, norepinephrine, vasopression, dopamine, dobutamine, neosynephrine, or milrinone). Following discontinuation of cardiopulmonary bypass, immediately before sternal closure, utilization and doses of any of the above vasopressors were recorded. Finally, at twenty-four hours following CABG procedure data on utilization and dose of vasopressor therapy were recorded,

**Evaluation of post-operative clinical outcomes**. Following CABG procedure these additional clinical parameters were recorded:
1. Evaluation for occurrence of post-operative cardiac arrhythmia's
2. Length of intubation post-operatively
3. Need for reintubation
4. Length of ICU stay
5. Length of hospital stay
6. Survival trends (including 6 month survival)

**Expected results and critique:** GLN therapy was used to improve cardiac function indices (particularly cardiac index) following discontinuation of cardiopulmonary bypass and at 24 hours, following CABG. In regard to use of vasopressor therapy, GLN therapy reduces the need for vasoactive drug use. Finally, GLN therapy decreases the time of intubation and ICU stay following CABG.

**Statistical Analysis:** Normality of distribution of data was checked by visual inspection of Q-Q plots. A log transformation was applied when necessary and checked again for normality. Comparison of the two groups were performed with one-way or repeated-measures ANOVA. ICU and Hospital length of stay and length of intubation were compared via Mann-Whitney U test. Ratios and ordinal data were compared with the two-sided Fisher's exact test. A p value < 0.05 was considered significant. The SPSS software package (version 10.07, SPSS Inc., Chicago IL) was used for all statistical analyses.

**Post-study Data analysis:** All collected data was tested for significance as previously described. The SPSS software package (version 10.07, SPSS Inc., Chicago IL) was used to calculate distribution statistics and for all statistical analyses. A p value of < 0.05 was considered significant.

### Example 2:

Glutamine (GLN) protects against endotoxin-induced mortality. Increased Interleukin-18 (IL-18) levels appear to play a critical role in cytokirie-induced organ failure and can be predictive of mortality in sepsis patients. Post-treatment with GLN attenuates pro-inflammatory (IL-18, IL-6, and cytokine expression, enhances lung heat shock protein 72 (HSP 72) expression, and improves survival following 2CLP. Sprague-Dawley rats (250-300g) underwent 2CLP and one hour following surgery a single dose of GLN (0.75 g/Kg) (n=18) or a balanced salt solution (control) (n=17) was administered. Survival was monitored for five days. In a separate group of animals, blood was drawn at multiple time points for analysis of cytokine (IL-18, IL-6 TNF-α and IL-10) expression. Lung tissue was harvested for analysis of HSP 72. GLN administration one hour following 2CLP significantly attenuated the expression of IL-18 at 12, 18, and 24 hours post-2CLP (p < 0.05). Expression of IL-6 and TNF-α was attenuated at six hours post-2CLP (p < 0.05). GLN enhanced lung HSP 72 expression versus control (p < 0.05) following 2CLP. Finally, GLN decreased the five-day mortality rate from 78% to 42% (p < .04) following 2CLP. The data indicates that GLN can markedly improve survival from 2CLP. Potential mechanisms for the protection include attenuation of pro-inflammatory cytokine expression and enhanced lung HSP 72 expression. Further, this is the first description of IL-18 expression in the 2CLP model. These data indicate that a single dose of GLN given as post-treatment can attenuate the systemic inflammatory response syndrome (SIRS) and prevent lethality from polymicrobial sepsis.

### Example 2:

Sepsis is known to be associated with an intrinsic derangement of tissue metabolism. Recent data indicates a major determinant of oxygen utilization and tissue metabolic function is cellular levels of NAD+. GLN can preserve ATP content and attenuate lactate accumulation following endotoxemic shock. The present study tests the hypothesis that GLN, given as post-treatment, preserves NAD+ levels and attenuates the metabolic dysfunction in lung and heart tissue following polymicrobial sepsis.

**Methods:** Male Sprague-Dawley rats (300-350 g) underwent cecal ligation and puncture (CLP) and one hour following surgery a single dose of GLN (0.75 g/Kg) (n=18) or a balanced salt solution (CONT) (n=17) was administered. Survival was monitored for five days. In a separate group of animals (n=4/group), lung and heart tissue was harvested 24 hours post-CLP and tissue metabolism was analyzed by ¹H-MRS and ³¹ P-MRS.

**Results:** GLN administration led to a significant improvement in NAD + content in both lung and heart tissue following CLP (p < 0.005). Further, GLN significantly attenuated the fall in lung tissue ATP/ADP ratio (p < 0.005), Creatine/phosphocreatine (p < 0.01), glucose (p < 0.01), and glutamine (p < .005). GLN administration led to a trend towards a decrease in lung lactate accumulation (p < 0.06). GLN-mediated attenuation of tissue metabolic dysfunction was associated with a significant decrease in the five day mortality rate post-CLP [78% (GLN) vs. 42% (CONT) (p < 0.04).

**Conclusions:** GLN administration can attenuate the metabolic dysfunction and fall in NAD+ observed in lung and heart tissue following sepsis induced via cecal ligation and puncture. The most significant effect was observed in lung tissue. The improved tissue metabolism is associated with increased survival. Possible explanations for the effect can include GLN manipulation of heat shock protein, attenuated PPAR activation, preserved tissue glutathione content, or via enhanced availability of metabolic substrate.

### Example 3:

A single dose of glutamine can improve heart function following ischemia and reperfusion injury. This is related, to glutamine's ability to prevent metabolic dysfunction following ischemia and reperfusion. This sort of glutamine dosing can improve outcomes from Cardiac diseases such as coronary artery disease, heart attacks, and cardiopulmonary bypass procedures.

Glutamine (given as alanyl-glutamine dipeptide) can improve outcomes from ischemia and reperfusion injury in the heart when given as a single dose. This was shown in a rat model of ischemia and reperfusion injury. It is likely that this is due to preservation of metabolic function and glutathione levels. Enhanced heat shock protein expression also plays a role.

A single dose of glutamine dipeptide (alanyl-glutamine) given to a rat 18 hours prior to myocardial injury can improve myocardial function following ischemia and reperfusion injury. This protection is due to preservation of metabolism and glutathione levels. A single dose of alanyl-glutamine is functions via preservation of tissue metabolism (ATP content exc.) and preservation of tissue glutathione levels.

The concept of a commonly occurring amino acid dipeptide, like alanyl-glutamine, as a single dose drug to improve outcomes from cardiac ischemia and reperfusion has never been examined by any other investigator.

The technology can be used to give alanyl-glutamine or other glutamine dipeptide to patients coming to the ER with heart attacks, patients coming for cardiac surgery, or patients with chronic angina. The benefit is enormous as this a benign and relatively cheap amino acid.

### Example 4:

No pharmacological agent has been found to be beneficial in the treatment of heatstroke. Previous research has indicated that enhanced heat shock protein 70 (HSP 70) expression can improve survival following experimental heat stroke. However, no clinically relevant enhancer of HSP 70 has been utilized in human trials. Intravenously administered glutamine (GLN) can enhance tissue HSP 70 expression in rodent models of sepsis and ischemia/reperfusion injury. The present tested whether administered GLN could enhance tissue HSP 70 expression and improve survival following heatstroke in the rat.

**Methods:** GLN (0.65 g/kg) (n=8) or a balanced salt solution (BSS) (n=8) (Control, vehicle GLN was dissolved in) was administered to Sprague-Dawley Rats (250-300 g) via gavage twice a day for five days prior to experimental heatstroke. Heatstroke was performed in anesthetized rats (ketamine/xylazine) by heating animals to 42 deg C (rectal temperature) for 30 minutes. Survival was analyzed for five days post-heatstroke. In a separate set of animals, HSP 70 and Heat Shock Factor-1 activation (HSF-1) were analyzed at 1-hour and 24-hours post-heatstroke in heart and lung tissue via western blot (n= 10/group). Statistical analysis was performed via Fischer's Exact Test for survival and ANOVA with Duncan grouping as post-hoc test for HSP-70 and HSF-1 expression.

**Results:** In control animals, heatstroke led to significant mortality ((5/8), 62.5%), whereas oral GLN therapy significantly reduced mortality ((1/8), 12.5%) (p < 0.003). All mortality occurred in the first 48 hours post-heatstroke. GLN administration also significantly enhanced HSP 70 expression 1 hour post-heatstroke in both heart and lung tissue (p < 0.01 for both tissues versus unheated animals and heated controls). A significant increase in HSP 70 expression was seen in heart and lung tissue at 24 hours in both GLN-treated and control animals versus unheated animals. However, no significant difference was seen in HSP 70 between GLN and control animals at 24 hours post-heatstroke. No change in HSF-1 activation (the transcription factor for HSP 70) was seen at either time point tested following heatstroke.

**Conclusions:** The results demonstrated that oral, *in vivo,* administration of GLN can improve survival following lethal heatstroke injury. The results also indicated for the first time that oral GLN can enhance tissue HSP 70 expression immediately post-heatstroke injury. The fact that there was a similar increase in HSP 70 at 24 hours post-heatstroke in both heated groups establishes that GLN can accelerate the expression of HSP 70 and that this is one mechanism of enhanced heatstroke survival via GLN. The fact that no change was seen in HSF-1 activation shows that GLN causes a more rapid translation of existing HSP 70 mRNA, rather then new HSP 70 gene activation. The data indicated that oral GLN is useful in prevention of mortality from heatstroke in at risk populations, such as athletes and soldiers.

### Example 5:

Pathways of sepsis-induced injury include inflammatory cytokine release, enhanced iNOS activity, and tissue metabolic dysfunction. The heat stress protein (HSP) pathway is known to play a significant role in the pathophysiology of sepsis and enhanced HSP activation can improve survival post-sepsis.

In Cecal-Ligation and Puncture (CLP) in the rat, a single dose of glutamine (GLN) post-initiation of CLP can enhance lung HSP expression, attenuate tissue injury pathways, and improve survival.

**Methods:** Male Sprague-Dawley rats were divided into 5 groups. Two groups were treated with 400 mg/kg of quercetin (Q) IP (HSP 70 inhibitor) 6 hours pre-CLP. CLP was performed and GLN (0.75 g/kg, IV) or balanced salt solution (BSS) was administered one-hour post-CLP (n=10 group). The next two groups received the same GLN + CLP (n=18) or BSS + CLP (n=17) treatment minus quercetin. The final group (CONT, n=6) received no treatment. Five-day survival was monitored. In separate studies, lung was harvested 24-hours post-CLP and tissue metabolism analyzed by 31 P- and 1 H-MRS. A final study analyzed blood for TNF- at 6 hours post-CLP and lung tissue 6 hours post-CLP for HSP-70, iNOS, eNOS, and activated heat shock factor-1 (HSF-1).

**Results:** A single dose of GLN post-sepsis enhanced lung HSF-1 activation and HSP70 expression. GLN led to a significant improvement in 5-day survival, attenuated lung iNOS, and plasma TNF- expression. GLN enhanced lung eNOS and improved lung ATP/ADP ratio, NAD+, and attenuated lactate accumulation. Inhibition of HSF-1 activation and subsequent HSP70 expression by Q abrogated GLNs survival benefit and blocked improvement in lung tissue metabolic dysfunction. No Q effect was noted on TNF- or NOS expression.

**Conclusions:** GLN's ability to enhance HSP activation and attenuate tissue metabolic dysfunction is a key mechanism in GLN's attenuation of sepsis mortality. Due to Q effects, GLN attenuation of TNF- and iNOS activity did not appear to be key mechanisms of GLNs beneficial effects.

### Example 6:

Elevated Serum Heat Shock Protein 72 (sHSP72) has been associated with increased survival after severe trauma. However, no clinically relevant enhancer of HSP72 has been found in humans. Glutamine (GLN) has been shown to enhance tissue HSP72 levels and improve survival post-sepsis and systemic inflammatory response (SIRS) in rodents. The present study was performed to determine if GLN could enhance sHSP72 levels and improve survival in experimental SIRS in the rat and to evaluate if GLN could enhance sHSP72 levels in patients with pancreatitis and indications of systemic inflammation.

**Methods:** sHSP72 was measured in Sprague-Dawley rats that underwent cecal ligation and puncture (CLP) and 1 hour post-CLP a single dose of GLN (given as ALA-GLN) (0.75 g/Kg) (n=4) or a balanced salt solution (BSS) (n=4) was given. A third group (CONT, n=4) received no treatment. Survival was monitored in a separate set of rats for 5 days. sHSP72 was measured in CONT rats and GLN/BSS rats 6 hours post-CLP. sHSP72 were measured via ELISA. sHSP72 was evaluated in patients with pancreatitis post-surgical debridement. Eligible patients were in ICU at enrollment with signs of systemic inflammation as measured by C-reactive protein > 10. Eligible patients required TPN > 7 days. Patients had a baseline sHSP72 drawn and were randomized to iv GLN (0.3 g/kg) (n=3) (given as ALA-GLN) or standard TPN (n=3). sHSP72 was drawn 7 days post-enrollment.

**Results:** GLN significantly enhanced sHSP72 (p < 0.05 vs. BSS) and survival (61 % (GLN) vs. 18% (BSS); p < 0.01) following experimental SIRS.

### Example 7:

The above *in vivo* and *in vitro* data have shown that the induction of the heat shock response can attenuate pro-inflammatory cytokine release. Overwhelming pro-inflammatory and immune responses are key features of septic shock and play an essential role in the pathogenesis of tissue damage, multiple organ system dysfunction, and ultimately death. Glutamine (given as a pharmacologic supplement) has previously been shown to improve survival, increase heat shock protein expression and attenuate the pro-inflammatory cytokine release in LPS treated animals, an effect that occurs without the complete inhibition of cytokine release or blockade of subsequent effects. Glutamine deficiency has also been shown to modulate *in vitro* cytokine, production in cultured blood mononuclear cells (PMBCs) and supplementation reduces interleukin (IL)-6 and IL-8 in cultured human duodenal tissue.

To date, no pharmacologic agent has been found to be beneficial in the treatment of heatstroke in humans. Heat stress and heat stroke have previously been shown to have a profound effect on circulating cytokine release and enhanced Heat Shock Protein 70 (HSP 70) can improve survival following experimental heat stroke.

The benefit of the pre-treatment of glutamine is enormous, as this is a benign and relatively inexpensive amino acid. One use for the pre-treatment of oral glutamine is the prevention of mortality from heat stroke in at-risk populations such as soldiers and athletes where subsequent heat stress to the body is inevitable.

The above data shows that pharmacologic glutamine supplementation is a potent enhancer of heat shock protein 72 (HSP 72) expression *in vitro* and *in vivo* experiments. Additionally, glutamine-treated peripheral blood mononuclear cells exhibited an increase in heat shock protein expression and had significant attenuation of tumor-necrosis alpha release. Specifically, rats orally pre-treated with glutamine for five consecutive days and subsequently exposed to experimental heat stroke had improved survival and increased Heat Shock Protein expression.

**Methods:** Eligible patients randomized to the glutamine (treatment) group as their initial treatment receives oral L-Glutamine at a dose of 0.65g/kg twice a day for five days prior to subsequent heat stress in a hot tub in the GCRC. Patients also took the L-glutamine on the morning before subsequent heat stress. Patients were then be placed in hot tub at a temperature of 40 degrees Celsius (+/-2 degrees C) for 30 minutes or until their oral temperature reaches 99.6 degrees F or above. The selected dose of L-Glutamine was based on the maximum dose that has been previously safely administered in humans. The dose has been tolerated well without adverse side effects even in critically ill patients.

After a period of 4 weeks for washout after the initial study (the half-life of glutamine is 6 hours), the patient returned and continued the same process with the placebo that consisted of a maltodextran powder. Patients that were randomized to take the placebo initially did so, then after a 2-week washout period returned to finish the L-Glutamine treatment arm of the study.

Blood was drawn from all patients (10 cc/time point) in order to assess cytokine response, heat shock protein expression, and glutamine levels throughout the course of the study. Baseline blood was taken after consent was obtained and the instructions of use of oral L-Glutamine/placebo were given. A blood sample was drawn on the morning prior to heat stress in the hot tub; after the last dose of L-Glutamine/placebo was given. Additional time points for blood draws included 1 hour, 6 hours, and 24 hours after patient was subjected to heat stress. Each time point is dependant upon when particular cytokines (TNF-alpha, IL-1, II-6, and IL-18) in the blood are being expressed during the pro-inflammatory cascade, however all cytokines were measured at each time point.

Ten ml samples of blood were drawn into EDTA tubes at baseline, after the last dose of L-Glutamine/placebo, 1, 6 and 24 hours. The tubes were centrifuged and plasma was separated for Glutamine and cytokine analysis. Samples were frozen and stored at 80°C. Additionally, five ml of blood were taken into red top tubes at baseline, after the last dose of L-Glutamine/placebo, 1, 6 and 24 hours for subsequent LPS stimulation of the blood.

Approximately 75 ml of blood were drawn from each subject during each arm the study in total. There was a washout period of one month, and then the patients had another 75 ml of blood drawn.

Analysis of Heat Shock Protein Expression:Serum Heat Shock Protein 70 (HSP 70) expression was analyzed using an enzyme-linked immunosorbent assay (ELISA) specific to human serum HSP 70. (Stressgen, Victoria BC, Canada) Assay was performed according to manufacturer's instruction. HSP 70, HO-1 and HSP 27 were also analyzed using Western blotting. Blood was drawn at 6 hours post heat stress and placed in blood collecting tubes containing protease inhibitor cocktail (Roche Molecular, Indianapolis, Indiana). Blood was then centrifuged for 3 minutes at 5000 revolutions/min, and supernatant containing plasma was removed and frozen at -80 degrees Celsius. Protein concentrations of samples were determined utilizing the Lowry method. Western Blotting was performed using 10% SDS PAGE gels in a standard fashion using 1X treatment buffer. Millipore membranes were blocked with 5% Blotto (5% weight: volume nonfat dry milk in phosphate buffered saline with 0.1% Tween). For HSP72 detection, blots were then incubated with a specific mouse monoclonal antibody, C92 (StressGen, Victoria, BC, Canada). Blots were then washed and incubated with a secondary goat anti-mouse HRPO antibody (Santa Cruz, CA) and developed using an enhanced chemiluminescence system. For control protein HSC73, a specific rat monoclonal antibody to constitutive HSC73 (StressGen) was utilized. For HO-1 detection the previously mentioned Western Blot technique was applied, and the membrane was incubated with a rabbit polyclonal antibody to HO-1 (StressGen).

### Analysis of Pro-inflammatory Cytokine Release:

**TNF-Alpha Detection:** TNF-Alpha concentrations were measured using an enzyme-linked immunosorbent assay (ELISA). Blood was drawn at 6 hours post heat stress and stimulated with LPS. TNF- Alpha concentrations were determined utilizing an ELISA kit for TNF-Alpha from Endogen (Woburn, Massachusetts). Results were then determined spectrophotometrically using a microplate reader (Thermo Lab Systems Opsys MR, Chantily, Virginia).

**IL-6 and IL-1 Detection:** Interleukin-6 and Interleukin-1 concentrations were measured using an enzyme-linked immunosorbent assay (ELISA). Blood was drawn at 6 hours post heat stress and stimulated with LPS. The blood was then analyzed utilizing ELISA kits for Rat IL-6 and IL-1 respectively from Research Diagnostics Inc. (Flanders, New Jersey). Results were then obtained spectrophotometrically using a microplate reader (Thermo Lab Systems Opsys MR, Chantily, Virginia).

**IL-18 Detection:** Blood was drawn at 24 hours post heat stress and stimulated with LPS. Interleukin-18 concentrations were detected using an ELISA kit as previously described from Biosource International (Camarillo, California).

### Example 8:

GLN's protective effects may be due to enhanced heat shock protein (HSPs) expression, specifically HSP 70. It has long been known that induction of HSP 70 in experimental animals via sublethal heating and/or arsenic is markedly protective against many forms of injury. However, a clinically relevant enhancer of HSP 70 that can safely and easily be given to human subjects has been lacking. GLN can enhance HSP 70 and other HSPs in cells, tissues, whole animals, and critically patients. The enhancement of HSP 70 via enhanced activation of the transcription factor responsible for HSP 70 expression (Heat Shock Factor-1 (HSF-1)) can protect cells and experimental animals from a wide variety of injuries and stresses. Finally, the administration of this safe and non-toxic amino acid to critically ill humans can enhance HSP-70 in the serum of these patients.

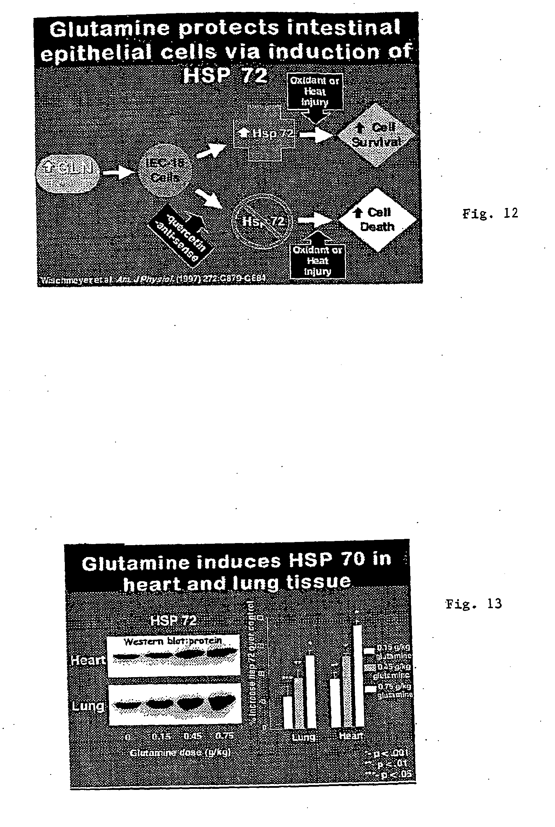

**Glutamine protection of cells from injury and stress is mediated via enhanced HSP 70 expression.** Initial experiments indicated that GLN protection of cells against heat and oxidant stress was due to enhanced expression of HSP 70. This was proven when blockade of HSP 70 expression via quercetin (inhibits HSF-1 binding) and via direct antisense inhibition of the HSP-70 gene (Figure 12).

**Glutamine protects against endotoxin-induced sepsis and enhances HSP 70 and HSP 25 in multiple tissues in experimental animals.** The next set of experiments assessed whether GLN could induce HSPs in whole animals. GLN's effects in both stressed and unstressed animals were evaluated. GLN can enhance HSP 70 and HSP 25 in the heart and lung tissue of unstressed rats (Figure 13).

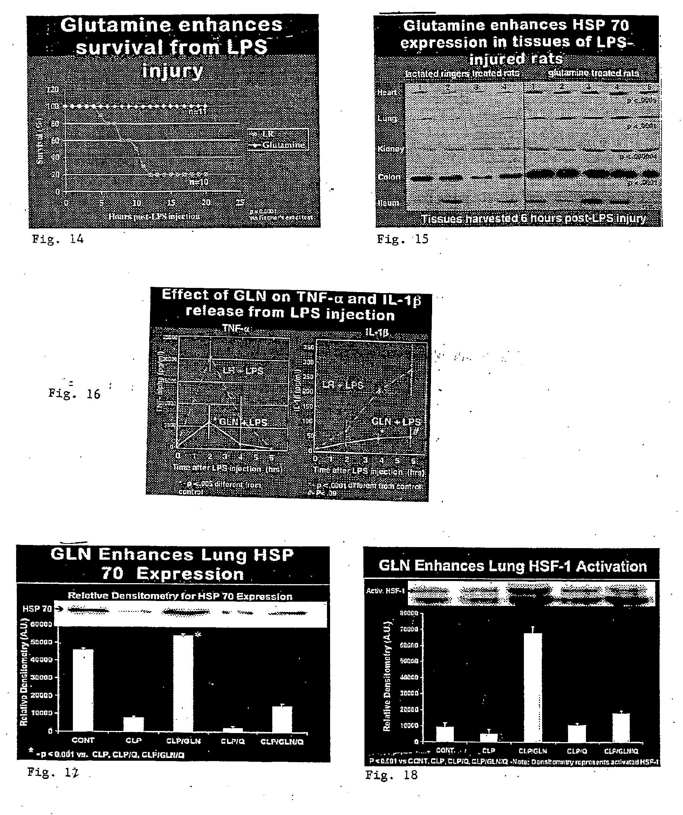

The effect of GLN on survival and HSP expression following endotoxemia was then evaluated. GLN was administered concurrently with lethal doses of endotoxin. GLN reduced mortality and enhanced HSP 70 and 23 following lethal endotoxemia (Figures 14 and 15).

Evaluation of pro-inflammatory cytokine release was also assessed following endotoxemia. GLN attenuated TNF-alpha and IL-1 beta following endotoxemia (Figure 16).

**GLN enhances survival following polymicrobial sepsis via induction of HSP 70 and enhanced activation of heat shock factor-1 (HSF-1).** As lethal endotoxemia is not a clinically relevant model of sepsis, the effect of post-treatment with GLN on lung HSP 70 expression, HSF-1 activation, and survival following polymicrobial sepsis and peritonitis in a clinically relevant model was assessed. Cecal ligation and puncture (CLP) in the rat was chosen as the appropriate model. The lung expression of HSP was focused on as previous data has indicated that adenoviral transfection of HSP 70 into the lungs of animals undergoing this same insult could enhance deficient HSP 70 expression and double survival. The animals underwent induction of peritonitis and sepsis and were treated 30 minutes later with GLN. GLN treatment markedly enhanced deficient lung HSP 70 expression and HSF-1 activation. GLN also markedly enhanced serum HSP 70 expression. The effect was blocked by administration of quercetin an inhibitor of HSP 70 expression (Signified by Q in the figures) (Figure 17 and 18).

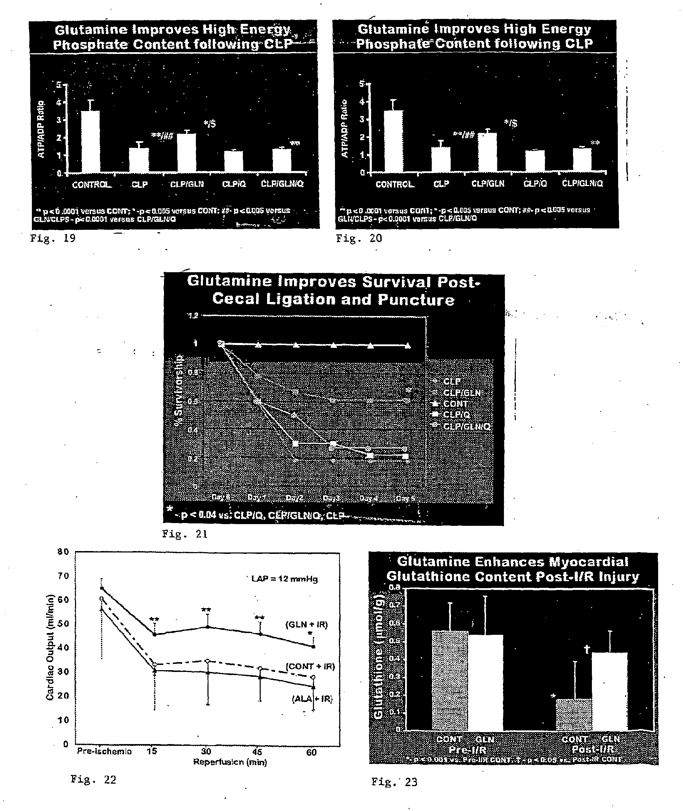

GLN also was able to attenuate tissue metabolic dysfunction following sepsis and peritonitis by nearly doubling ATP/ADP ratio and NAD+/NADH levels in lung tissues (Figures 19 and 20). The effect was inhibited by the HSP 70 inhibitor quercetin (Signified by Q below).

Finally, GLN was able to significantly improve survival following peritonitis and polymicrobial sepsis (Figure 21). The effect was inhibited by the administration of quercetin. The data indicates that GLN can able to protect against peritonitis, sepsis, and infection following injury.

**Single dose of GLN enhances myocardial tissue metabolism, glutathione content, and improves myocardial function after ischemia-reperfusion injury.** The effect of GLN on cardiac function following myocardial ischemia and reperfusion injury (I/R), an injury that mimics a myocardial infarction-like injury, was assessed. To assess GLN's effect a single dose of glutamine was administered 18 hours prior to 15 minutes of no flow ischemia utilizing a rat working heart model. GLN treated animals had significant improvement in post-I/R cardiac output (Figure 22). Significant improvement in parameters of myocardial tissue metabolism was observed. The results included improved ATP/ADP ratio, NAD+/NADH content, and decreased myocardial tissue lactate accumulation. Further, GLN improved myocardial glutathione content (Figure 23) post I/R. The data indicates that GLN could protect patients from organ I/R injury and/or myocardial I/R injury, such as myocardial infarction.

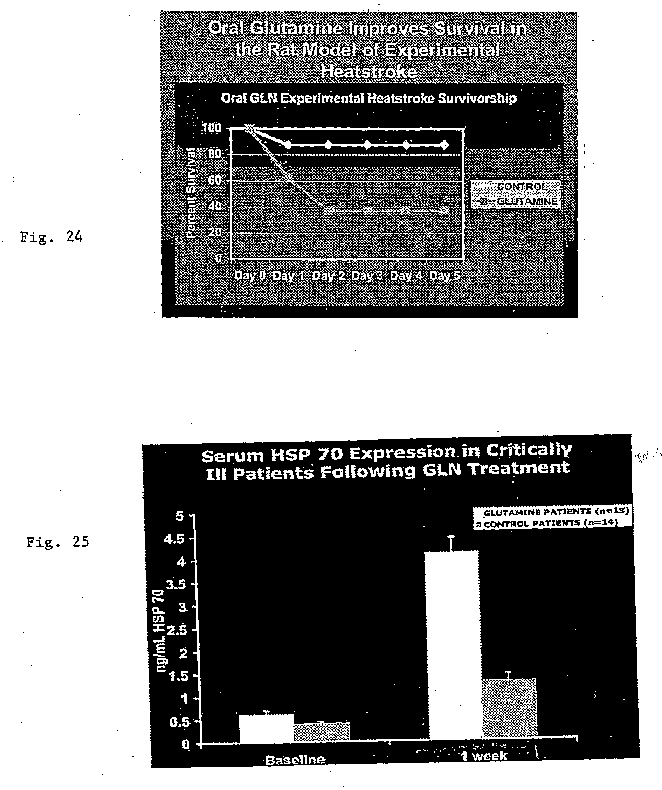

**GLN enhances survival and HSP 70 expression following experimental heatstroke injury.** It was next determined if orally administered GLN was protective against mortality from lethal heatstroke. GLN's effect on HSP 70 expression in this rat model was also evaluated. Five days of oral GLN prior to lethal heatstroke injury significantly reduced mortality (Figure 24) and enhanced HSP 70 expression in heart, lung, and colon tissue. Orally administered GLN is able to attenuate morbidity and mortality in soldiers and other patients at risk for heatstroke.

**GLN enhances serum HSP 70 levels in serum of critically ill patients.** It was next determine whether GLN could enhance HSP 70 in critically ill patients. To determine this, GLN or an iso-nitrogenous control solution was administered intravenously to a mixed group of critically ill patients in a surgical ICU setting. GLN led to a four-fold enhancement of serum HSP levels versus iso-nitrogenous control solution in these patients (Figure 25). Serum HSP 70 levels in the polymicrobial sepsis/peritonitis model previously described in the rat (CLP model) were examined. An approximate four-fold increase in serum HSP 70 levels was found, which was correlated with a doubling of survival. The data is particularly important, as previous research in trauma patients has shown that enhanced serum HSP 70 levels are correlated with increased survival in severely traumatized patients.

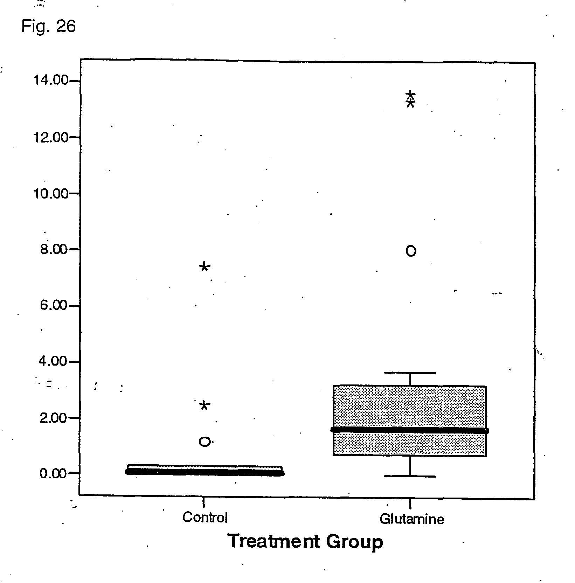

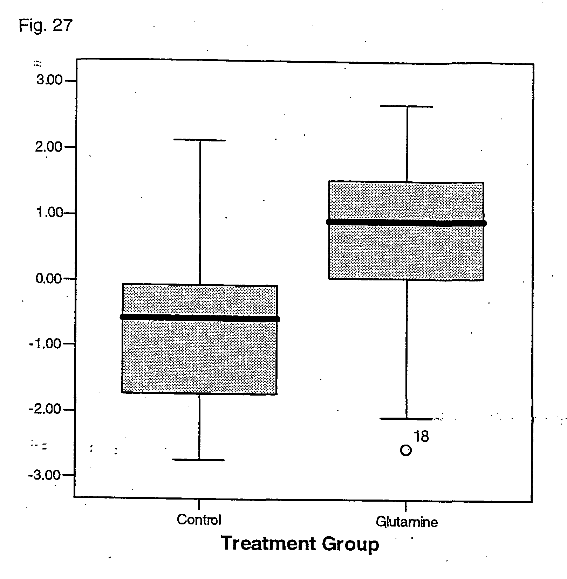

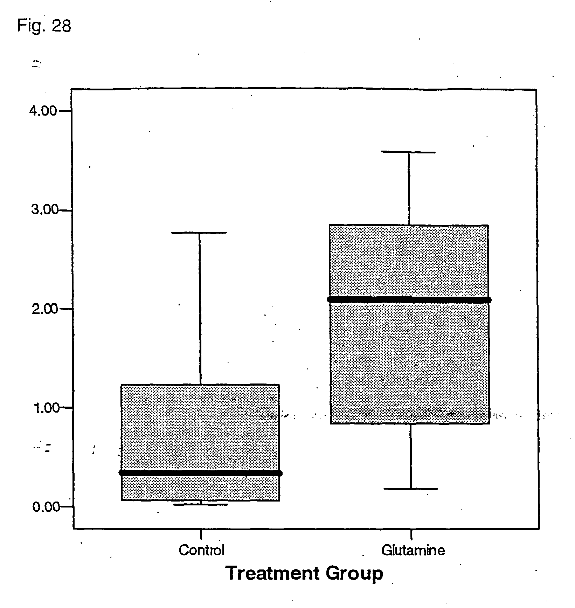

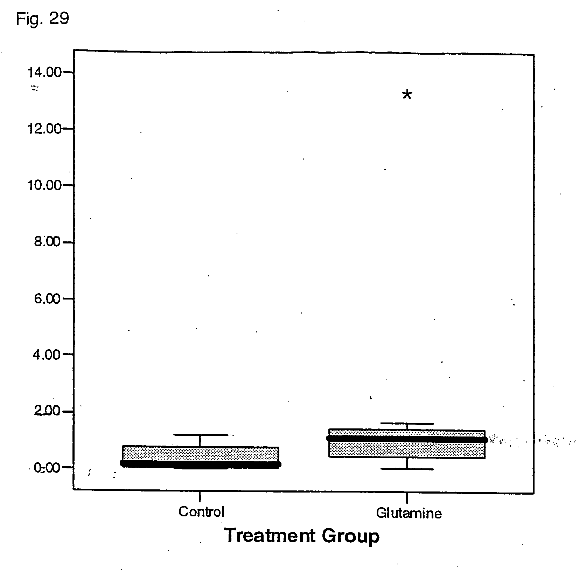

Further, as shown in Figures 26-29 and Tables 1-10, HSP-70 levels at 1-week were 2.82 units higher in patients who received Glutamine compared to control patients (95% Cl: -0.01 to 5.64, p = .050). The change in HSP-70 levels from baseline to 1-week was 2.60 units greater in patients who received Glutamine compared to control patients (95% Cl: -0.09 to 5.29, p = .057). HSP-70 levels at 1-week were 3.71 times higher in patients who received Glutamine compared to controls (95% Cl: 1.15 to 11.85, p = .029). In other words, the rate of increase in HSP-70 levels over 1-week was 3.40 times greater in patients who received Glutamine compared to controls (95% Cl: 1.50 to 7.70, p = .005).

Throughout this application, various publications, including United States patents, are referenced by author and year, and patents, by number. Full citations for the publications are listed below. The disclosures of these publications and patents in their entireties are hereby incorporated by reference into this application in order to more fully describe the state of the art to which this invention pertains.

The invention has been described in an illustrative manner, and it is to be understood that the terminology that has been used is intended to be in the nature of words of description rather than of limitation.

Obviously, many modifications and variations of the present invention are possible in light of the above teachings. It is, therefore, to be understood that within the scope of the described invention, the invention can be practiced otherwise than as specifically described.

**Table 1**

| Baseline characteristics for glutamine and control subjects, all diagnoses. | | | |
|---|---|---|---|
| | | *Glutamine* (*n=15*) | *Control* (*n=14*) |
| Age | | 53.2 ± 15.0 | 52.4±14.1 |
| Gender (%) | | | |
| | 1 | 53.3 | 78.6 |
| | 2 | 46.7 | 21.4 |
| Diagnosis (%) | | | |
| | 1 | 53.3 | 71.4 |
| | 2 | 13.3 | 14.3 |
| | 3 | 20.0 | 7.1 |
| | 4 | 13.3 | 7.1 |
| Baseline CRP | | 19.8±11.0 | 17.6 ± 7.8 |
| BMI | | 31.4±7.3 | 28.8 ± 7.6 |
| Hospital Day of Entry | | | |
| Baseline HSP-70 | | 0.61±1.05 | 0.39 ± 0.37 |

**Table 2**

| Baseline characteristics for glutamine and control subjects, non-pancreatic diagnoses. | | | |
|---|---|---|---|
| | | *Glutamine* (*n=15*) | *Control* (*n=14*) |
| Age | | 59.9 ± 14.4 | 58.3±15.3 |
| Gender (%) | | | |
| | 1 | 57.1 | 75.0 |
| | 2 | 42.9 | 25.0 |
| Diagnosis (%) | | | |
| | 2 | 28.6 | 50.0 |
| | 3 | 42.9 | 25.0 |
| | 4 | 28.6 | 25.0 |
| Baseline CRP | | 23.6±13.0 | 20.2±10.9 |
| BMI | | 32.1±6.8 | 29.6 ±11.6 |
| Hospital Day of Entry Baseline HSP-70 | | 0.83±1.48 | 0.25±0.35 |

**Table 3**

| Comparison of HSP-70 levels for glutamine and control subjects, non-pancreatic diagnoses. | | | | |
|---|---|---|---|---|
| | *Glutamine (n = 7*) | *Control* (*n = 4)* | *p-value^{T}* | *NP p-value^{t}* |
| Baseline HSP-70 | 0.83 ± 1.48 | 0.25 ± 0.35 | .355 | .230 |
| 1-week HSP-70 | 3.43 ± 5.17 | 0.65 ± 0.53 | .206 | .230 |
| HSP-70 change from baseline | 2.60 ± 4.77 | 0.40 ± 0.56 | .271 | .315 |
| LN(Baseline HSP-70) | -1.43 ± 1.58 | -2.01 ± 1.18 | .504 | .230 |
| LN(1-week HSP-70) | 0.20 ± 1.70 | -0.90 ± 1.32 | .269 | .230 |
| LN(1-wk HSP / Baseline HSP) | 1.63 ± 1.21 | 1.12 ± 1.32 | .550 | .412 |

| | | | | |
|---|---|---|---|---|
| *^{T}*Independent samples unequal variances t test *^{t}* Non-parametric test: Mann-Whitney U test | | | | |

**Table 4**

| Comparison of HSP-70 levels in glutamine and control subjects, all diagnoses. | | | | |
|---|---|---|---|---|
| | *Glutamine* (*n = 15*) | *Control* (*n = 14*) | *p-value^{T}* | *NP p-value^{t}* |
| Baseline HSP-70 | 0.61 ± 1.05 | 0.39 ± 0.37 | .457 | .983 |
| 1-week HSP-70 | 4.09 ± 4.70 | 1.27 ± 2.24 | **.050** | **.026** |
| HSP-70 change from baseline | 3.48 ± 4.53 | 0.88 ± 2.01 | **.057** | **.010** |
| LN(Baseline HSP-70) | -1.37 ± 1.26 | -1.46 ±1.11 | .849 | .983 |
| LN(1-week HSP-70) | 0.56 ± 1.63 | -0.75 ± 1.42 | **.029** | **.026** |
| LN(1-wk HSP / Baseline HSP) | 1.93 ± 1.23 | 0.71 ± 0.89 | **.005** | **.006** |

| | | | | |
|---|---|---|---|---|
| *^{T}*Independent samples unequal variances t test *^{t}* Non-parametric test: Mann-Whitney U test | | | | |

**Table 5**

| Comparison of outcomes for glutamine and control subjects, all diagnoses. | | | | |
|---|---|---|---|---|
| | *Glutamine (n* = *15)* | *Control (n = 14^{¶})* | *p-value^{T}* | *NP p-value^{t}* |
| Number of infections | 1.87 ± 2.59 | 3.00 ± 3.19 | .305 | .331 |
| One or more infections (%) | 60.0 % | 71.4% | .518 | .700 |
| Number | 0.20 ± 0.41. | 0.57 ± 0.94 | .190 | .400 |
| One or more bacterial infections (%) | 20.0% | 35.7% | .344 | .427 |
| Length of stay (days) | 29.73 ± 14.46 | 33.29 ± 22.18 | .617 | .880 |
| ICU days | 15.73 ± 16.18 | 17.38 ± 22.49 | .828 | .650 |
| Ventilator days | 12.07 ± 15.25 | 15.77 ± 21.57 | .611 | .751 |
| Maximum Total Bilirubin (*units??*) | 1.23 ± 0.67 | 2.01 ± 1.95 | .174 | .847 |
| Maximum Total Creatinine (*units??*) | 1.26 ± 0.89 | 1.47 ± 1.51 | .653 | .949 |

| | | | | |
|---|---|---|---|---|
| *^{T}*Independent samples unequal variances t test or Pearson Chi-Square test *^{t}* Non-parametric test: Mann-Whitney U test or Fisher's exact test *^{¶}* Sample size is 13 patients for ICU days and ventilator days. | | | | |

**Table 6**

| Comparison of outcomes for glutamine and control subjects, non-pancreatic diagnoses. | | | | |
|---|---|---|---|---|
| | *Glutamine* (*n = 7*) | *Control* (*n = 4^{¶}*) | *p-value^{T}* | *NP p-value*^{t} |
| Number of infections | 1.43 ± 1.40 | 4.25 ± 2.36 | **.091** | **.073** |
| One or more infections (%) | 57.1 % | 100% | .125 | .212 |
| Number of bacterial infections | 0.0 ± 0.0 | 1.0 ± 1.41 | .252 | .230 |
| One ore more bacterial infection (%) | 0 % | 50 % | **.039** | **.109** |
| Length of stay (days) | 25.29 ± 6.18 | 31.25 ± 17.59 | .554 | .412 |
| ICU days | 16.71 ± 7.65 | 26.00 ± 24.52 | .582 | .517 |
| Ventilator days | 12.86 ± 9.03 | 25.33 ± 25.50 | .488 | .517 |
| Maximum Total Bilirubin (*units??*) | 1.70 ± 0.70 | 4.23 ± 2.19 | .102 | .164 |
| Maximum Total Creatinine (*units??*) | 1.80 ± 1.07 | 2.78 ± 2.48 | .499 | .648 |

| | | | | |
|---|---|---|---|---|
| *^{T}*Independent samples unequal variances t test or Pearson Chi-Square test *^{t}* Non-parametric test: Mann-Whitney U test or Fisher's exact test *^{¶}* Sample size is 3 patients for ICU days and ventilator days. | | | | |

**Table 7**

| Spearman Rank Correlations between HSP-70 and clinical outcomes among patients treated with Glutamine, all diagnoses (n=15). | | | |
|---|---|---|---|
| *Outcome* | *1-week HSP-70* | *Change in HSP-70* | *HSP-70 Ratio* |
| Number of infections | -0.401 | **-0.466** | -0.384 |
| | p=.139 | **p=.080** | p=.158 |
| | | | |
| Number of bacterial | -0.309 | -0.309 | -0.424 |
| infections | p=.263 | p=.263 | p=.115 |
| | | | |
| Length of stay | -0.082 | -0.123 | -0.206 |
| | p=.771 | p=.661 | p=.462 |
| | | | |
| ICU days | -0.438 | **-0.615** | **-0.649** |
| | p=.103 | **p=.015** | **p =.009** |
| | | | |
| Ventilator days | -0.327 | **-0.483** | **-0.551.** |
| | p = .235 | **p=.068** | **p =.033** |
| | | | |
| Maximum Total Bilirubin | -0.213 | -0.371 | -0.025 |
| | p=.445 | P=.173 | p =.929 |
| | | | |
| Maximum Total | -0.327 | **-0.476** | -0.230 |
| Creatinine | p=.235 | **p=.073** | p=.410 |

**Table 8**

| Spearman Rank Correlations between HSP-70 and clinical outcomes among patients with non-pancreatic diagnoses (n =11). | | | |
|---|---|---|---|
| *Outcome* | *1-week HSP-70* | *Change in HSP-70* | *HSP-70 Ratio* |
| Number of infections | -0.443 | -0.485 | -0.333 |
| | p=.172 | p=.130 | p=.318 |
| | | | |
| Number of bacterial | -0.418 | **-0.580** | **-0.666** |
| infections | p=.201 | **p = .062** | **p = .027** |
| | | | |
| Length of stay | -0.245 | -0.418 | -0.518 |
| | p=.467 | p=.201 | p=.102 |
| | | | |
| ICU days | -0.438 | **-0.693** | **-0.669** |
| | p =.206 | **p = .026** | p = **.035** |
| | | | |
| Ventilator days | -0.280 | -0.511 | -0.498 |
| | p=.434 | p=.132 | p= .143 |
| | | | |
| Maximum Total Bilirubin | -0.351 | -0.437 | -0.428 |
| | p=.290 | p = .179 | p = .189 |
| | | | |
| Maximum Total | -0.218 | -0.236 | -0.200 |
| Creatinine | p=.519 | p=.484 | p = .555 |

| | | | |
|---|---|---|---|
| NOTES: There were no relationships between HSP-70 levels and outcomes in the total sample of 29 patients. | | | |

**Table 9**

| Pearson and Spearman Rank Correlations between HSP-70 and clinical outcomes among patients treated with Glutamine, all diagnoses (n=15). | | | | |
|---|---|---|---|---|
| *Outcome* | *1-week HSP-70.* | | *Change in HSP-70* | |
| | *Pearson* | *Spearman* | *Pearson* | *Spearman* |
| Number of infections | -0.359 | -0.401 | -0.387 | **-0.466** |
| | p=.189 | p=.139 | p=.154 | **p=.080** |
| | | | | |
| Number of bacterial infections | -0.253 | -0.309 | -0.264 | -0.309 |
| | p=.263 | p=.263 | p=.341 | p=.263 |
| | | | | |
| Length of stay | -0.127 | -0.082 | -0.162 | -0.123 |
| | p=.652 | p=.771 | p=.563 | p=.661 |
| | | | | |
| ICU days | -0.373 | -0.438 | **-0.447** | **-0.615** |
| | p =.1.71 | p =.103 | **p=094.** | **p=015** |
| | | | | |
| Ventilator days | -0.306 | -0.327 | -0.394 | **-0.483** |
| | p=.267 | p=.235 | p=.146 | **p=.068** |
| | | | | |
| Maximum Total Bilirubin | -0.292 | -0.213 | -0.349 | -0.371 |
| | p=.290 | p=.445 | p=.202 | p=.173 |
| | | | | |
| Maximum Total Creatinine | -0.283 | -0.327 | -0.271 | **-0.476** |
| | p=.307 | p=.235 | p=.329 | **p =.073** |

**Table 10**

| Pearson and Spearman Rank Correlations between HSP-70 and clinical outcomes among patients with non-pancreatic diagnoses (n =11). | | | | |
|---|---|---|---|---|
| *Outcome* | *1-week HSP-70* | | *Change in HSP-70* | |
| | *Pearson* | *Spearman* | *Pearson* | *Spearman* |
| Number of infections | -0.416 | -0.443 | -0.412 | -0.485 |
| | p=.203 | p=.172 | p=.208 | p=.130 |
| | | | | |
| Number of bacterial infections | -0.182 | -0.418 | -0.197 | **-0.580** |
| | p=.593 | p=.201 | p=.561 | **p=.062** |
| | | | | |
| Length of stay | 0.092 | -0.245 | 0.087 | -0.418 |
| | p=.788 | p=.467 | p=.799 | p=.201 |
| | | | | |
| ICU days | -0.381 | -0.438 | -0.247 | **-0.693** |
| | p=.278 | p=.206 | p=.219 | **p=.026** |
| | | | | |
| Ventilator days | -0.319 | -0.280 | -0.378 | -0.511 |
| | p = .369 | p=.434 | p=.282 | p=.132 |
| | | | | |
| Maximum Total Bilirubin | -0.349 | -0.351 | -0.352 | -0.437 |
| | p=.292 | p=.290 | p=.289 | p=.179 |
| | | | | |
| Maximum Total Creatinine | -0.271 | -0.218 | -0.224 | -0.236 |
| | p=.420 | p=.519 | p=.508 | p=.484 |

| | | | | |
|---|---|---|---|---|
| NOTES: There were no relationships between HSP-70 levels and outcomes in the total sample of 29 patients. | | | | |

## Claims

1. A di-pepfide
said di-peptide being selected from the group consisting of alanyl-glutamine, glycyl-glutamine, leucyl-glutamine, valyl-glutamine, isoleucyl-glutamine, and cysteinyl-glutamine
for use in the treatment or prevention of an acute or chronic illness, disease, or injury; the prevention of ischemia or reperfusion injury; the protection of organs for transplantation; attenuating inflammation or pro-inflammatory cytokine expression in states of health or disease; the prevention or treatment of heat stress, heat stroke, or any other temperature related stress or injury; improving tissue metabolism; and treating sepsis;
wherein the illness, disease or injury is one of lung injury, acute respiratory distress syndrome, an autoimmune illness, arthritis, osteoarthritis, rheumatoid arthritis, Lupus, multiple sclerosis, fibromyalgia, Chrohn's disease, ulcerative colitis, irritable bowel syndrome, a heart attack, cardiac surgery, chronic angina, coronary artery disease, peritonitis and systemic inflammatory response syndrome, wherein the medicament is arranged to be delivered in an intravenous bolus dose.

2. A di-peptide
said di-peptide being selected from the group consisting of alanyl-glutamine, glycyl-glutamine, leucyl-glutamine, valyl-glutamine, isoleucyl-glutamine, and cysteinyl-glutamine for use in the treatment of local and systemic inflammatory diseases and autoimmune conditions, in order to protect organs for transplantation, wherein the medicament increases the expression of any heat shock protein or heat shock factor 1 and wherein the medicament is arranged to be delivered in an intravenous bolus dose.

3. A di-peptide
said di-peptide being selected from the group consisting of alanyl-glutamine, glycyl-glutamine, leucyl-glutamine, valyl-glutamine, isoleucyl-glutamine, and cysteinyl-glutamine for use in inhibiting skin/epithelial damage due to UV radiation, bum injury, aging, or any other type of radiation injury, wherein the medicament increases the expression of any heat shock protein or heat shock factor 1 and wherein the medicament is arranged to be delivered in a single intravenous bolus dose.

4. A di-peptide
said di-peptide being selected from the group consisting of alanyl-glutamine, glycyl-glutamine, leucyl-glutamine, valyl-glutamine, isoleucyl-glutamine, and cysteinyl-glutamine for use in inhibiting skin or mucosal injury from chemotherapy or therapeutic radiation, wherein the medicament increases the expression of any heat shock protein or heat shock factor 1 and wherein the medicament is arranged to be delivered as an intravenous bolus dose.

## Patentansprüche

1. Ein Dipeptid,
jenes Dipeptid ausgewählt aus der Gruppe bestehend aus Alanylglutamin, Glycylglutamin, Leucylglutamin, Valylglutamin, Isoleucylglutamin und Cysteinylglutamin zur Verwendung in der Behandlung oder Vorbeugung einer akuten oder chronischen Erkrankung, Krankheit oder Verletzung; der Vorbeugung von Ischämie oder Reperfusionsverletzung; dem Schutz von Organen für die Transplantation; dem Abschwächen von Entzündung oder proinflammatorischer Zytokinexpression in Zuständen von Krankheit oder Gesundheit; der Vorbeugung oder Behandlung von Hitzestress, Hitzschlag oder jeglichem anderen temperaturbezogenem Stress oder Verletzung; der Verbesserung des Gewebestoffwechsels; und der Behandlung von Sepsis;
wobei die Erkrankung, Krankheit oder Verletzung eines von Lungenverletzung, akutes Atemnotsyndrom, eine Autoimmunkrankheit, Arthritis, Osteoarthritis, rheumatoide Arthritis, Lupus, multiple Sklerose, Fibromyalgie, Chrohns Krankheit, Colitis ulcerosa, Reizdarmsyndrom, ein Herzanfall, Herzchirurgie, chronische Angina, Herzkranzgefäßexkrankung, Peritonitis und systemisches Entzündungsreaktionssyndrom ist, wobei das Medikament vorbereitet ist, in einer intravenösen Bolusdosis verabreicht zu werden.

2. Ein Dipeptid,
jenes Dipeptid ausgewählt aus der Gruppe bestehend aus Alanylglutamin, Glycylglutamin, Leucylglutamin, Valylglutamin, Isoleucylglutamin und Cysteinylglutamin zur Verwendung in der Behandlung von lokalen und systemischen Entzündungserkrankungen und Autoimmunleiden, um Organe für die Transplantation zu schützen, wobei das Medikament die Expression von irgendeinem Hitzeschockprotein oder Hitzeschockfaktor 1 erhöht und wobei das Medikament vorbereitet ist, in einer intravenösen Bolusdosis verabreicht zu werden.

3. Ein Dipeptid,
jenes Dipeptid ausgewählt aus der Gruppe bestehend aus Alanylglutamin, Glycylglutamin, Leucylglutamin, Valylglutamin, Isoleucylglutamin und Cysteinylglutamm zur Verwendung in der Hemmung von Haut-oder Epithelschaden auf Grund von UV-Strahlung, Verbrennungsverletzung, Altern oder jeglichen anderen Art von Strahlungsverletzung, wobei das Medikament die Expression von irgendeinem Hitzeschockprotein oder Hitzeschockfaktor 1 erhöht und wobei das Medikament vorbereitet ist, in einer intravenösen Bolusdosis verabreicht zu werden.

4. Ein Dipeptid,
jenes Dipeptid ausgewählt aus der Gruppe bestehend aus Alanylglutamin, Glycylglutamin, Leucylglutamin, Valylglutamin, Isoleucylglutamin und Cysteinylglutamm zur Verwendung in der Hemmung von Haut- oder Schleimhautverletzung durch Chemotherapie oder therapeutische Bestrahlung, wobei das Medikament die Expression von irgendeinem Hitzeschockprotein oder Hitzeschockfaktor 1 erhöht und wobei das Medikament vorbereitet ist, als eine intravenöse Bolusdosis verabreicht zu werden.

## Revendications

1. Dipeptide,
ledit dipeptide étant choisi dans le groupe comprenant l'alanyl-glutamine, la glycyl-glutamine, la leucyl-glutamine, la valyl-glutamine, l'isoleucyl-glutamine et la cystéinyl-glutamine,
pour son utilisation dans le traitement ou la prévention d'une maladie, d'un trouble ou d'une lésion aiguë ou chronique ; la prévention de l'ischémie ou de lésions de perfusion répétée ; la protection d'organes destinés à la transplantation ; l'atténuation d'une inflammation ou de l'expression des cytokines pro-inflammatoires dans des états sain ou de maladie ; la prévention ou le traitement d'un stress thermique, d'une insolation ou d'un quelconque autre stress ou trouble lié à la température ; l'amélioration du métabolisme tissulaire ; et le traitement de la sepsie ;
dans lequel la maladie, le trouble ou la lésion est l'un parmi une lésion pulmonaire, un syndrome de détresse respiratoire aiguë, une maladie auto-immune, l'arthrite, l'ostéo-arthrite, l'arthrite rhumatoïde, le lupus, la sclérose en plaques, la fibromyalgie, la maladie de Crohn, la colite ulcéreuse, le syndrome du côlon irritable, une crise cardiaque, une chirurgie cardiaque, une angine chronique, une maladie coronarienne, une péritonite et un syndrome de réaction inflammatoire systémique, dans lequel le médicament est conçu pour être délivré dans un bolus intraveineux.

2. Dipeptide,
ledit dipeptide étant choisi dans le groupe comprenant l'alanyl-glutamine, la glycyl-glutamine, la leucyl-glutamine, la valyl-glutamine, l'isoleucyl-glutamine et la cystéinyl-glutamine, pour son utilisation dans le traitement de maladies inflammatoires locales et systémiques et de conditions auto-immunes, afin de protéger les organes destinés à la transplantation, dans lequel le médicament augmente l'expression d'une protéine de choc thermique ou d'un facteur de choc thermique 1 quelconque et dans lequel le médicament est conçu pour être délivré dans un bolus intraveineux.

3. Dipeptide,
ledit dipeptide étant choisi dans le groupe comprenant l'alanyl-glutamine, la glycyl-glutamine, la leucyl-glutamine, la valyl-glutamine, l'isoleucyl-glutamine et la cystéinyl-glutamine, pour son utilisation dans l'inhibition d'une lésion cutanée/épithéliale due au rayonnement UV, d'une brûlure, d'un vieillissement ou de n'importe quel autre type de lésion due au rayonnement, dans lequel le médicament augmente l'expression d'une protéine de choc thermique ou d'un facteur de choc thermique 1 quelconque et dans lequel le médicament est conçu pour être délivré dans un seul bolus intraveineux.

4. Dipeptide,
ledit dipeptide étant choisi dans le groupe comprenant l'alanyl-glutamine, la glycyl-glutamine, la leucyl-glutamine, la valyl-glutamine, l'isoleucyl-glutamine et la cystéinyl-glutamine, pour son utilisation dans l'inhibition d'une lésion cutanée ou de muqueuse issue d'un rayonnement chimiothérapeutique ou thérapeutique, dans lequel le médicament augmente l'expression d'une protéine de choc thermique ou d'un facteur de choc thermique 1 quelconque et dans lequel le médicament est conçu pour être délivré sous la forme d'un bolus intraveineux.
